# EUROPEAN PATENT SPECIFICATION

(11) **EP 4 157 451 B1**
(45) Date of publication and mention of the grant of the patent: **04.12.2024**
(21) Application number: 21818840.7
(22) Date of filing: 02.06.2021
(51) Int. Cl.: A61K 31/137, A23L 27/14, A23F 3/34, A61P 27/00, A61P 25/00, A61K 9/08, A61K 9/06, A61K 47/32, A61K 47/10, A61K 9/00, A61P 9/02, A61K 31/522, A61K 31/165, A61K 45/06

(54) **SUBLINGUAL FORMULATION FOR HYPOTENSION AND SYNCOPE**
SUBLINGUALE FORMULIERUNG FÜR HYPOTONIE UND SYNKOPEN
FORMULATION SUBLINGUALE CONTRE L'HYPOTENSION ET LA SYNCOPE

(30) Priority: 02.06.2020 US 202063033360 P
(43) Date of publication of application: 05.04.2023
(73) Proprietor: Hamdan, Mohamed Hussein, Middleton, WI 53562 (US); Brignole, Michele, 16041 Borzonasca (IT); Guieu, Regis, 13009 Marseille (FR); Deharo, Jean-Claude, 13008 Marseille (FR); Michelet, Pierre, 13009 Marseille (FR)
(72) Inventor: Hamdan, Mohamed Hussein, Middleton, WI 53562 (US); Brignole, Michele, 16041 Borzonasca (IT); Guieu, Regis, 13009 Marseille (FR); Deharo, Jean-Claude, 13008 Marseille (FR); Michelet, Pierre, 13009 Marseille (FR)
(74) Representative: Algemeen Octrooi- en Merkenbureau B.V.
(86) International application number: PCT/US2021/035363
(87) International publication number: WO 2021/247647

(56) References cited:
- US-A- 5 919 210
- US-A1- 2008 008 770
- US-A1- 2009 148 546
- US-A1- 2009 155 392
- ANONYMOUS: "New Energy Spray Uses Capsaicin to Deliver Caffeine for Sustained Mental Focus", PRWEB, 6 November 2009 (2009-11-06), XP055879918, Retrieved from the Internet <URL:https://www.prweb.com/pdfdownload/3168724.pdf> [retrieved on 20220117]
- KAZUO FURUKAWA: "Prevention of postprandial hypotension-related syncope by caffeine in a patient with long-standing diabetes mellitus", ENDOCRINE JOURNAL, vol. 67, no. 6, 1 January 2020 (2020-01-01), JP, pages 585 - 592, XP093158128, ISSN: 0918-8959, DOI: 10.1507/endocrj.EJ19-0370
- PAUL HUTSON: "Safety, Pharmacokinetic, and Pharmacodynamic Study of a Sublingual Formula for the Treatment of Vasovagal Syncope", DRUGS IN R&D, vol. 22, no. 1, 12 February 2022 (2022-02-12), pages 61 - 70, XP093158129, ISSN: 1174-5886, Retrieved from the Internet <URL:https://link.springer.com/article/10.1007/s40268-021-00378-9/fulltext.html> DOI: 10.1007/s40268-021-00378-9
- ANONYMOUS: "New Energy Spray Uses Capsaicin to Deliver Caffeine for Sustained Mental Focus", PRWEB, 6 November 2009 (2009-11-06), XP055879918, Retrieved from the Internet <URL:https://www.prweb.com/pdfdownload/3168724.pdf>
- WRIGHT C. I., E THAKORE: "Syncope: dietary advice to help manage the symptoms of syncope", INTEGRATIVE FOOD, NUTRITION AND METABOLISM, vol. 3, no. 5, 11 November 2016 (2016-11-11), XP055879920, DOI: 10.15761/IFNM.1000165
- BRAND: SINUS BUSTER: "SiCap Spray Awake Energy Spray - 1 oz", 3 February 2011 (2011-02-03), UK, pages 1 - 2, XP009532941, Retrieved from the Internet <URL:https://www.amazon.co.uk/SiCap-Spray-Awake-Energy/dp/B000HI6QL2> [retrieved on 20210805]

## Description

### CROSS-REFERENCE TO RELATED APPLICATIONS

This application claims the benefit of U.S. Prov. Appl. 63/033,360, filed June 2, 2020.

### FIELD OF THE INVENTION

The present invention relates to compositions, formulations and devices for preventing syncope and treating or preventing hypotension associated with diseases and disorders, and in particular to the use of combinations of capsaicin, caffeine, and α-agonists to prevent syncope.

### BACKGROUND OF THE INVENTION

Syncope is defined as transient loss of consciousness associated with inability to maintain postural tone with rapid and spontaneous recovery (Shen et al 2017). The presumed cause is cerebral hypoperfusion. The prevalence depends on the population being evaluated and has been reported to be as high as 41% with recurrent syncope occurring in 13.5% (Lamb et al 1960). In the emergency department, syncope accounts for 0.8% to 2.4% of all visits (Olde Nordkamp et al 2009; Day et al 1982; Morichetti and Astorino 1998). Hospital costs associated with the inpatient evaluation of syncope exceed $2.4 billion per year in the United States (Sun 2013).

Vasovagal syncope (VVS) is the most common type of syncope. It is defined as syncope occurring after prolonged standing or with exposure to emotional stress, pain, or medical settings, and is associated with features such as diaphoresis, warmth, nausea, and pallor. Patients with VVS often experience fatigue following the event (Sheldon et al 2015). Vasovagal syncope is very common affecting up to 42% of woman and 32% of men by the age of 60 (Ganzeboom et al 2006; Serletis et al 2006; Goyal Parag 2016). The outcome in patients with VVS is benign; however, the 1-year recurrence rate is 25-35% leading to significant impairment in quality of life including injuries and loss of employment (Sumner et al 2010).

The mechanism of VVS is reflex-mediated triggered by various afferent inputs to the brain. The efferent response includes vagal activation leading to cardio-inhibition (CI) and brady-arrhythmias, and sympathetic withdrawal leading to a decrease in systemic vascular resistance and vasodilatation (VD) (Morillo et al 1997; Mosqueda-Garcia 2000). The result is cerebral hypoperfusion. The hemodynamic pattern, i.e. cardioinhibitory, vasodepressive, or both, is independent of the trigger evoking reflex syncope. In patients where the trigger is prolonged standing, a reduction in preload alone is thought to be sufficient to reduce cerebral hypoperfusion without the need for sympathoinhibition (Fu and Levine 2014; Jardine et al 2002; Cook and Convertino 2002). Adenosine is an ATP derivative that impacts strongly the cardiovascular system through the activation of its membrane receptors A1R, A2AR, A2BR, or A3R, based upon their pharmacological properties. Activation of A1R leads to sinus bradycardia or AV block, while activation of A2AR leads to vasodilation (Iwamoto 1994; Ponnoth 2009; Arsyad 2016; Kleppish 1995; Shryock and Belardinelli 1997).

Endogenous adenosine is implicated in most forms of reflex syncope. VVS patients have high adenosine plasma levels, high A2AR expression and special A2AR gene polymorphism (Saadjian et al 2002 and 2009, Deharo et al 2012). Theophylline, a nonspecific antagonist of adenosine receptors, is a useful daily treatment of some forms of reflex syncope (Brignole et al 2016).

Caffeine has been proposed as a useful compound for the prevention of postprandial hypotension-related syncope. Orally administered caffeine by means of a black coffee drink, improved a patient's postprandial hypotensive episodes by slowing the sympathetic system through the gradual elevation of plasma noradrenaline (Furukawa et al., "Prevention of postprandial hypotension-related syncope by caffeine in a patient with long-standing diabetes mellitus", Endocrine Journal vol. no. 67,, pp.: 585-592).

Caffeine drinks are considered for advice in patients with vasovagal syncope, due to the small and acute rise in blood pressure lead by caffeine, which counteracts the small decreases in arterial pressure seen in syncope patients (Wright C.I., Thakore E, "Syncope: dietary advice to help manage the symptoms of syncope", Integrative Food, Nutrition and Metabolism, vol. no. 3(5), pp.: 436-437.

Capsaicin has been disclosed to deliver sustained doses of caffeine in a sublingual spray for a sustained energy delivery. A sublingual dose of small amounts of caffeine produces big effects with big results and no side effects (Anonymous, "New Energy Spray Uses Capsaicin to deliver Caffeine for Sustained Mental Focus" PRWeb 6 November 2009, XP055879918, retrievable from URL:https://www.prweb.com/pdfdownload/3168724.pdf).

While several drugs are indicated in the treatment of VVS, what is needed in the art is a treatment of an impending syncopal attack.

### SUMMARY OF THE INVENTION

The invention is set out as encompassed in the appended set of claims. The present invention relates to composition, formulations and devices for use in preventing syncope, and in particular to combinations of capsaicin, caffeine, and α-agonists for use in preventing syncope.

Accordingly, in some preferred embodiments, the present invention provides compositions for use in treating or preventing a disease or condition selected from the group consisting of syncope, vasovagal reaction in blood donors, dialysis-induced hypotension, and symptomatic orthostatic hypotension, comprising: an effective amount per dose of capsaicin; and an effective amount per dose of caffeine; wherein the combined effective amounts of the capsaicin and caffeine are effective to treat or prevent syncope, vasovagal reaction in blood donors, dialysis-induced hypotension, or symptomatic orthostatic hypotension when administered sublingually to a subject in need thereof; and wherein the compositions further comprise an effective amount per dose of an α-agonist.

In some preferred embodiments, the effective amount per dose of capsaicin is from 0.1 to 10 mg. In some preferred embodiments, the effective amount per dose of capsaicin is from 0.3 to 5 mg. In some preferred embodiments, the effective amount per dose of capsaicin is from 0.5 to 1.5 mg. In some preferred embodiments, the effective amount per dose of caffeine is from 50 to 400 mg. In some preferred embodiments, the effective amount per dose of caffeine is from 100 to 300 mg. In some preferred embodiments, the effective amount per dose of caffeine is from 150 to 250 mg.

In some preferred embodiments, the α-agonist is selected from the group consisting of phenylephrine and etilefrine (ethylphenylephrine). In some preferred embodiments, the effective amount per dose of the α-agonist is from 1 to 100 mg. In some preferred embodiments, the effective amount per dose of the α-agonist is from 2 to 70 mg. In some preferred embodiments, the effective amount per dose of caffeine of the α-agonist is from 5 to 50 mg.

In some preferred embodiments, the compositions further comprise one of more pharmaceutically acceptable carriers. In some preferred embodiments, the pharmaceutically acceptable carrier is compatible with sublingual administration. In some preferred embodiments, the pharmaceutically acceptable carrier is selected from the group consisting of an aqueous carrier, a gelling agent and combinations thereof. In some preferred embodiments, the aqueous carrier is selected from the group consisting of water, an alcohol, and combinations thereof. In some preferred embodiments, the gelling agent is selected from the group consisting of polyvinylpyrrolidone, polyethylene glycol and combinations thereof. In some preferred embodiments, the polyethylene glycol is selected from the group consisting of polyethylene glycol 3350, polyethylene glycol 400, and combinations thereof. In some preferred embodiments, the composition is an aqueous solution. In some preferred embodiments, the composition is an aqueous gel. In some preferred embodiments, the composition further comprises a flavoring agent. In some preferred embodiments, the composition further comprises a stabilizing agent. In some preferred embodiments, the composition further comprises a coloring agent.

In other preferred embodiments, the present invention provides sublingual drug delivery formulations for use in treating or preventing a disease or condition selected from the group consisting of syncope, vasovagal reaction in blood donors, dialysis-induced hypotension, and symptomatic orthostatic hypotension comprising: an effective amount per dose of capsaicin; an effective amount per dose of caffeine; and one or more pharmaceutically acceptable carriers compatible with sublingual administration; wherein the combined effective amounts of the capsaicin and caffeine are effective to treat or prevent syncope, vasovagal reaction in blood donors, dialysis-induced hypotension, or symptomatic orthostatic hypotension when administered sublingually to a subject in need thereof; wherein the composition further comprises an effective amount per dose of an alpha-agonist.

In some preferred embodiments, the effective amount per dose of capsaicin is from 0.1 to 10 mg. In some preferred embodiments, the effective amount per dose of capsaicin is from 0.3 to 5 mg. In some preferred embodiments, the effective amount per dose of capsaicin is from 0.5 to 1.5 mg. In some preferred embodiments, the effective amount per dose of caffeine is from 50 to 400 mg. In some preferred embodiments, the effective amount per dose of caffeine is from 100 to 300 mg. In some preferred embodiments, the effective amount per dose of caffeine is from 150 to 250 mg.

In some preferred embodiments, the α-agonist is selected from the group consisting of phenylephrine and etilefrine (ethylphenylephrine). In some preferred embodiments, the effective amount per dose of the α-agonist is from 1 to 100 mg. In some preferred embodiments, the effective amount per dose of the α-agonist is from 2 to 70 mg. In some preferred embodiments, the effective amount per dose of the α-agonist is from 5 to 50 mg.

In some preferred embodiments, the pharmaceutically acceptable carrier is selected from the group consisting of an aqueous carrier, a gelling agent and combinations thereof. In some preferred embodiments, the aqueous carrier is selected from the group consisting of water, an alcohol, and combinations thereof. In some preferred embodiments, the gelling agent is selected from the group consisting of polyvinylpyrrolidone, polyethylene glycol and combinations thereof. In some preferred embodiments, the polyethylene glycol is selected from the group consisting of polyethylene glycol 3350, polyethylene glycol 400, and combinations thereof. In some preferred embodiments, the formulation is an aqueous solution. In some preferred embodiments, the formulation is an aqueous gel. In some preferred embodiments, the formulation further comprises a flavoring agent. In some preferred embodiments, the formulation further comprises a stabilizing agent. In some preferred embodiments, the formulation further comprises a coloring agent.

In some preferred embodiments, the present invention provides a composition, formulation or device as described above for use in preventing or inhibiting a disease or condition selected from the group consisting of syncope, vasovagal reaction in blood donors, dialysis-induced hypotension, and symptomatic orthostatic hypotension in a subject in need thereof.

### BRIEF DESCRIPTION OF THE DRAWINGS

**FIG. 1** provides data showing effects of sublingual administration of Caffeine (200mg) and Capsaicine (50µL/0.5%) dissolved in 1 mL of ethanol on systolic blood pressure (SBP) in a patient suffering from impending VVS. The drugs were administered at the beginning of prodromes (T0), leading to an increase in SBP from 65 mmHg to 155 mmHg after 1 minute that persisted for all the duration of tilt test procedure (T20). Symptoms disappeared and syncope did not occur.
**FIG. 2** provides data showing the kinetics of caffeine plasma level in 5 patients suffering from vasovagal syncope and administered with caffeine + capsaicin solubilized in ethanol.
**FIGs. 3A** and **3B** provide graphs of blood pressure and heart rate over time after administration of CPC.
**FIGs. 4A** and **4B** provide graphs of capsaicin blood levels over time after CPC administration.
**FIGs. 5A** and **5B** provide graphs of PE blood levels over time after CPC administration.
**FIGs. 6A** and **6B** provide graphs of caffeine blood levels over time after CPC administration.

### DEFINITIONS

As used herein, the term "subject" refers to any animal including, but not limited to, humans, non-human primates, bovines, equines, felines, canines, pigs, rodents (e.g., mice), and the like. The terms "subject" and "patient" may be used interchangeably, wherein the term "patient" generally refers to a human subject seeking or receiving treatment or preventative measures from a clinician or health care provider.

As used herein, the term "effective amount" refers to the amount of a compound (e.g., a compound as described herein) sufficient to effect beneficial or desired results. An effective amount can be administered in one or more administrations, applications or dosages and is not limited to or intended to be limited to a particular formulation or administration route.

As used herein, the term "co-administration" refers to the administration of at least two agent(s) (e.g., a compound having a structure presented above or elsewhere described herein) or therapies to a subject. In some embodiments, the co-administration of two or more agents/therapies is concurrent. In other embodiments, a first agent/therapy is administered prior to a second agent/therapy. Those of skill in the art understand that the formulations and/or routes of administration of the various agents/therapies used may vary. The appropriate dosage for co-administration can be readily determined by one skilled in the art. In some embodiments, when agents/therapies are co-administered, the respective agents/therapies are administered at lower dosages than appropriate for their administration alone. Thus, co-administration is especially desirable in embodiments where the co-administration of the agents/therapies lowers the requisite dosage of a known potentially harmful (e.g., toxic) agent(s).

As used herein, the term "pharmaceutical composition" refers to the combination of an active agent with a carrier, inert or active, making the composition especially suitable for diagnostic or therapeutic use in vivo, in vivo or ex vivo.

As used herein, the term "pharmaceutically acceptable carrier" refers to any of the standard pharmaceutical carriers, such as a phosphate buffered saline solution, water, emulsions (e.g., such as an oil/water or water/oil emulsions), and various types of wetting agents. The compositions also can include stabilizers and preservatives. For examples of carriers, stabilizers and adjuvants see (e.g., Martin, Remington's Pharmaceutical Sciences, 15th Ed., Mack Publ. Co., Easton, Pa. [1975]).

As used herein, the term "instructions for administering said composition/formulation to a subject," and grammatical equivalents thereof, includes instructions for using the compositions contained in a kit or device for the treatment of conditions (e.g., providing dosing, route of administration, decision trees for treating physicians for correlating patient-specific characteristics with therapeutic courses of action).

### DETAILED DESCRIPTION OF THE INVENTION

The present invention relates to compositions, formulations and devices for use in preventing syncope and treating or preventing hypotension associated with diseases and disorders, and in particular to the use of combinations of capsaicin, caffeine, and α-agonists for use in preventing syncope.

Accordingly, in some preferred embodiments, the compositions of the present invention for use to be administered to subjects in need of treatment for or related to syncope. In some preferred embodiments, the composition or formulation comprises effective amounts of caffeine, capsaicin and an alpha-agonist to relieve, reduce, inhibit or prevent the incidence of syncope in a subject in need thereof.

The composition and formulations of the present invention comprise capsaicin. Capsaicin (8-methyl-N-vanillyl-trans 6-nonenamide), is an alkaloid found naturally in many peppers that can induce painful stimuli via the activation of vanilloid receptors localized on C fibers (Smutzer et al 2016). It is contemplated that in the compositions and formulations of the present invention, capsaicin acts in two ways. First, capsaicin acts as a local vasodilator and therefore can facilitate the absorption of other agents, for example, phenylephrine and caffeine (see below). Second, capsaicin administration has local painful effects that elicit sympathetic activation. Indeed, through the emotional reaction to the perceived pain, it is contemplated that capsaicin increases heart rate and blood pressure, thus counteracting the hypotension/bradycardia reflex of VVS (Nordin and Fagius 1995). Furthermore, the anticipation of discomfort from the capsaicin may by itself increase sympathetic activity as a conditioned effect even before the dose is administered.

The compositions and formulations of the present invention further comprise caffeine. Caffeine is a nonspecific adenosine receptor antagonist. Caffeine blocks both A1 and A2A receptors, leading to an increase in heart rate and blood pressure (Riksen et al 2011).

The compositions for use of the present invention further comprise an alpha-agonist. Suitable alpha-agonists include, but are not limited to, phenylephrine and etilefrine (ethylphenylephrine). Phenylephrine and etilefrine (ethylphenylephrine) have powerful vasoconstrictive properties when delivered intravenously. In a placebo-controlled tilt study (Raviele et al 2005), the intra-atrial injection of a bolus of phenylephrine (1 mg/1 ml) at the time of impending syncope resulted in an immediate marked increase in blood pressure, which interrupted VVS in most patients. However, phenylephrine was unable to counteract the vagally-induced bradycardia of the reflex. Similarly, intravenous injection of etilefrine was successful in aborting impending VVS in 87% of cases as opposed to 20% with placebo (Ammirati et al 2000). The effect of the drugs occurred mostly within 20 seconds.

The present invention is not limited to any particular mechanism of action. Indeed, an understanding of the mechanism of action is not necessary to practice the present invention. Nevertheless, the combination of capsaicin, caffeine, and an alpha-agonist was selected based on a synergistic benefit from the combination, particularly in the context of providing a composition or formulation that works within seconds to suppress VVS.

In some preferred embodiments, it is contemplated the subject will self-administer the composition or formulation to abort or prevent syncope (i.e., provide effective protection against syncope occurrence). Thus, the instant invention allows for improving both health conditions and quality of life of millions of patients worldwide appears feasible and represents a new approach in the treatment of the common problem of syncope. It is further contemplated that the instant invention can eliminate the need for daily drug intake in patients with recurrent VVS and thus represent a solution to an unmet need in a large number of patients.

The compositions and formulations also provide a secondary beneficial effect. It is contemplated that there is a prolonged presence of one or more of the mixture up to several hours after oral administration. Thus, the instant compositions and formulations are effective for use in prophylaxis for VVS when used periodically during the day, or intermittently in high-risk syncope situations.

The compositions and formulations of the present invention are not limited to for use in therapy and prophylaxis of VVS. Indeed, the compositions and formulations of the present invention find prophylactic and therapeutic use for other diseases and disorders associated with hypotension, including blood donors suffering from vasovagal reactions, hemodialysis patients suffering from dialysis induced hypotension, and patients with symptomatic orthostatic hypotension (especially in cases where attempts have been made to address reversible causes).

*Blood donors:* Each year, an estimated 6.8 million people in the U.S. donate blood. The overall prevalence of vasovagal reactions (VVRs) in blood donors is estimated to be 1.4-7% (Blood Reviews 2012, 26, 33-42). The non-return rate for subsequent donations is 45% in first-time donors who experienced VVRs compared with 18% in donors who did not, with similar differences in repeat donors (Blood Transfusion 2014, 12 (Suppl. 1), s28-s36). The occurrence of VVRs was reduced in donors who received caffeine (125 or 250 mg) compared with those who received placebo (RR 0.42, 95% CI 0.21-0.85; p=0.02) (Health Psychology 1999, 18, 403-409). It is contemplated that administration of the compositions and formulations of the present invention will reduce the severity of VVRs in subjects who experience these symptoms, and the incidence of VVRs in repeat donors. Accordingly, in some preferred embodiments, the compositions of the present invention are administered to blood donors in need of treatment for vasovagal reactions. In some preferred embodiments, the composition or formulation comprises effective amounts of caffeine, capsaicin and an alpha-agonist to relieve, reduce, inhibit or prevent the incidence of vasovagal reaction in a blood donor.

*Hemodialysis Patients:* There are approximately 300,000 patients on chronic hemodialysis in the US with approximately 45M sessions per year. Hypotension during hemodialysis occurs in 6-27% of patients with a 20% incidence being most cited (Hemodial Int 2014; 18:415). While several factors could lead to hypotension during dialysis, it usually occurs in the absence of an acute serious condition. Suggested contributors include: Autonomic dysfunction particularly in diabetics (Am J Nephrol 1991; 11: 123-126); Release of adenosine during organ ischemia (J Am Soc Nephrol 1994; 4:1987); Increased synthesis of endogenous vasodilators such as nitric oxide (Ann Intern Med 1995; 123:35); and Insufficient increase in vasoconstrictors such as vasopressin (Nephrol Dial Transplant 1995; 10:1421-1427). It is contemplated that administration of the formulations and compositions described herein in patients with hemodialysis-induced hypotension reduces the magnitude of BP drop and thus premature termination of hemodialysis. Accordingly, in some preferred embodiments, the compositions of the present invention are administered to hemodialysis patients in need of treatment for dialysis-induced hypotension. In some preferred embodiments, the composition or formulation comprises effective amounts of caffeine, capsaicin and an alpha-agonist to relieve, reduce, inhibit or prevent the incidence of dialysis-induced hypotension.

*Orthostatic Hypotension:* The prevalence of orthostatic hypotension is 5-30% depending on age and type of population studied (Clin Auton Res 18 Suppl 1: 8-13; N Engl J Med 358: 615-624). Many conditions could lead to orthostatic hypotension including volume depletion, medications, and autonomic failure. Up to 40% of patients have no definite cause (Arch Intern Med. 1994;154(14):1620.). Current treatments include Florinef, Midodrine and Droxidopa (J Am Coll Cardiol, 70 (2017), pp. e39-e1 10). It is contemplated that administration of the compositions and formulations described herein will reduce the orthostatic drop in BP and thus risk of syncope and falls in patients with symptomatic orthostatic hypotension. Accordingly, in some preferred embodiments, the compositions of the present invention are administered to subjects in need of treatment for symptomatic orthostatic hypotension. In some preferred embodiments, the composition or formulation comprises effective amounts of caffeine, capsaicin and an alpha-agonist to relieve, reduce, inhibit or prevent the incidence of symptomatic orthostatic hypotension.

Thus, the compositions and formulations of the present invention comprise effective amounts of capsaicin, caffeine and an alpha-agonist. In some preferred embodiments, the effective amounts may be described as an effective amount per dose. A medical device of the present invention may therefore comprise a single dose or multiple doses containing effective amounts of the agent. In some preferred embodiments, the medical device, which may for example be a syringe or other container comprising a plunger or a pump spray device, is calibrated to deliver an effective dose by activating the delivery mechanism, for example, by depressing a plunger or pump.

In some preferred embodiments, the effective amount per dose of capsaicin in the composition or formulation is from 0.1 to 10 mg. In some preferred embodiments, the effective amount per dose of capsaicin in the composition or formulation is from 0.3 to 5 mg. In some preferred embodiments, the effective amount per dose of capsaicin in the composition or formulation is from 0.5 to 1.5 mg. In some preferred embodiments, the effective amount per dose of caffeine in the composition or formulation is from 50 to 400 mg. In some preferred embodiments, the effective amount per dose of caffeine in the composition or formulation is from 100 to 300 mg. In some preferred embodiments, the effective amount per dose of caffeine in the composition or formulation is from 150 to 250 mg. In some preferred embodiments, the effective amount per dose of the α-agonist in the composition or formulation is from 1 to 100 mg. In some preferred embodiments, the effective amount per dose of the α-agonist in the composition or formulation is from 2 to 70 mg. In some preferred embodiments, the effective amount per dose of the α-agonist in the composition or formulation is from 5 to 50 mg.

In some preferred embodiments, the compositions in formulations of the present invention contain one of more pharmaceutically acceptable carriers. In some preferred embodiments, the pharmaceutically acceptable carrier is compatible with sublingual administration. In some preferred embodiments, the pharmaceutically acceptable carrier is selected from the group consisting of an aqueous carrier, a gelling agent and combinations thereof. In some preferred embodiments, the aqueous carrier is selected from the group consisting of water, an alcohol, and combinations thereof. In some preferred embodiments, the gelling agent is selected from the group consisting of polyvinyl-pyrrolidone, polyethylene glycol and combinations thereof. In some preferred embodiments, the polyethylene glycol is selected from the group consisting of polyethylene glycol 3350, polyethylene glycol 400, and combinations thereof. In some preferred embodiments, the composition or formulation is an aqueous solution. In some preferred embodiments, the composition or formulation is an aqueous gel. In some preferred embodiments, the compositions further comprise one or more of a flavoring agent, a stabilizing agent, and a coloring agent.

The compositions and formulations of the present invention are not limited to the foregoing embodiments. Accordingly, in some preferred embodiments, the effective amounts or dosages of the capsaicin, caffeine, and an alpha-agonist are combined with one or more additional agents to form pharmaceutical compositions. Pharmaceutical compositions may be formulated in a conventional manner using one or more physiologically acceptable carriers including excipients and auxiliaries, which facilitate processing of the active compounds into preparations, which can be used pharmaceutically. Proper formulation is dependent upon the route of administration chosen. Additional details about suitable excipients for pharmaceutical compositions described herein may be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pa. 1975; Liberman, H. A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999).

A pharmaceutical formulation, as used herein, refers to a mixture of capsaicin, caffeine, and alpha-agonist as described herein, with other chemical components, such as carriers, stabilizers, diluents, dispersing agents, suspending agents, thickening agents, and/or excipients. The pharmaceutical formulation facilitates administration of the compound to an organism. In practicing the treatment or use provided herein, therapeutically effective amounts of compounds described herein are administered in a pharmaceutical composition to a mammal having a disease, disorder, or condition to be treated or prevented, such as syncope. In some embodiments, the mammal is a human. A therapeutically effective amount can vary widely depending on the severity of the disease, the age and relative health of the subject, the potency of the compound used and other factors. Furthermore, the capsaicin, caffeine, and alpha-agonist can further be used in combination with one or more additional therapeutic agents as components of mixtures (as in combination therapy).

The pharmaceutical formulations described herein can be administered to a subject by multiple administration routes, including but not limited to, sublingual, oral, buccal, nasal or transdermal routes. Moreover, the pharmaceutical compositions described herein, which include capsaicin, caffeine, and an alpha-agonist, can be formulated into any suitable dosage form, including but not limited to, aqueous oral dispersions, liquids, gels, syrups, elixirs, slurries, suspensions, aerosols, fast melt formulations, effervescent formulations and capsules.

Liquid formulation dosage forms for oral administration can be aqueous suspensions selected from the group including, but not limited to, pharmaceutically acceptable aqueous oral dispersions, emulsions, solutions, elixirs, gels, and syrups. See, e.g., Singh et al., Encyclopedia of Pharmaceutical Technology, 2nd Ed., pp. 754-757 (2002).

The aqueous suspensions and dispersions described herein can remain in a homogenous state, as defined in The USP Pharmacists' Pharmacopeia (2005 edition, chapter 905), for at least 4 hours. The homogeneity should be determined by a sampling method consistent with regard to determining homogeneity of the entire composition. In one embodiment, an aqueous suspension can be re-suspended into a homogenous suspension by physical agitation lasting less than 1 minute. In another embodiment, an aqueous suspension can be re-suspended into a homogenous suspension by physical agitation lasting less than 45 seconds. In yet another embodiment, an aqueous suspension can be re-suspended into a homogenous suspension by physical agitation lasting less than 30 seconds. In still another embodiment, no agitation is necessary to maintain a homogeneous aqueous dispersion.

Pharmaceutical formulations including capsaicin, caffeine, and an alpha-agonist as described herein may be manufactured in a conventional manner, such as, by way of example only, by means of conventional mixing, dissolving, levigating, emulsifying, encapsulating or entrapping processes.

In some preferred embodiments, compositions and formulations provided herein may also include one or more preservatives to inhibit microbial activity. Suitable preservatives include quaternary ammonium compounds such as benzalkonium chloride, cetyltrimethylammonium bromide and cetylpyridinium chloride.

The pharmaceutical formulations described herein can include capsaicin, caffeine, and an alpha-agonist as described herein, and one or more pharmaceutically acceptable additives such as a compatible carrier, binder, filling agent, suspending agent, flavoring agent, sweetening agent, disintegrating agent, dispersing agent, surfactant, lubricant, colorant, diluent, solubilizer, moistening agent, plasticizer, stabilizer, penetration enhancer, wetting agent, anti-foaming agent, antioxidant, preservative, or one or more combination thereof.

Suitable diluents for use in the formulations described herein include, but are not limited to, sugars (including lactose, sucrose, and dextrose), polysaccharides (including dextrates and maltodextrin), polyols (including mannitol, xylitol, and sorbitol), cyclodextrins and the like.

Suitable wetting agents for use in the formulations described herein include, for example, oleic acid, glyceryl monostearate, sorbitan monooleate, sorbitan monolaurate, triethanolamine oleate, polyoxyethylene sorbitan monooleate, polyoxyethylene sorbitan monolaurate, quaternary ammonium compounds, sodium oleate, sodium lauryl sulfate, magnesium stearate, sodium docusate, triacetin, vitamin E TPGS and the like.

Suitable surfactants for use in the formulations described herein include, for example, sodium lauryl sulfate, sorbitan monooleate, polyoxyethylene sorbitan monooleate, polysorbates, polaxomers, bile salts, glyceryl monostearate, copolymers of ethylene oxide and propylene oxide, and the like.

Suitable gelling and/or suspending agents for use in the formulations described here include, but are not limited to, polyvinylpyrrolidone, e.g., polyvinylpyrrolidone K12, polyvinylpyrrolidone K17, polyvinylpyrrolidone K25, or polyvinylpyrrolidone K30, polyethylene glycol, e.g., the polyethylene glycol can have a molecular weight of about 300 to 7000, vinyl pyrrolidone/vinyl acetate copolymer (S630), sodium carboxymethylcellulose, methylcellulose, hydroxy-propylmethylcellulose, polysorbate-80, hydroxyethylcellulose, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like.

Suitable antioxidants for use in the formulations described herein include, for example, e.g., butylated hydroxytoluene (BHT), sodium ascorbate, and tocopherol.

There is considerable overlap between additives used in the formulations described herein. Thus, the above-listed additives should be taken as merely exemplary, and not limiting, of the types of additives that can be included in formulations of the pharmaceutical compositions described herein.

The pharmaceutical formulations described herein may include sweetening agents such as, but not limited to, acacia syrup, acesulfame K, alitame, anise, apple, aspartame, banana, Bavarian cream, berry, black currant, butterscotch, calcium citrate, camphor, caramel, cherry, cherry cream, chocolate, cinnamon, bubble gum, citrus, citrus punch, citrus cream, cotton candy, cocoa, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, monoammonium glyrrhizinate, maltol, mannitol, maple, marshmallow, menthol, mint cream, mixed berry, neohesperidine DC, neotame, orange, pear, peach, peppermint, peppermint cream, raspberry, root beer, rum, saccharin, safrole, sorbitol, spearmint, spearmint cream, strawberry, strawberry cream, stevia, sucralose, sucrose, sodium saccharin, saccharin, aspartame, acesulfame potassium, mannitol, talin, sucralose, sorbitol, swiss cream, tagatose, tangerine, thaumatin, tutti fruitti, vanilla, walnut, watermelon, wild cherry, wintergreen, xylitol, or any combination of these flavoring ingredients, e.g., anise-menthol, cherry-anise, cinnamon-orange, cherry-cinnamon, chocolate-mint, honey-lemon, lemon-lime, lemon-mint, menthol-eucalyptus, orange-cream, vanilla-mint, and mixtures thereof.

In some embodiments, the pharmaceutical formulations described herein can be self-emulsifying drug delivery systems (SEDDS). Emulsions are dispersions of one immiscible phase in another, usually in the form of droplets. Generally, emulsions are created by vigorous mechanical dispersion. SEDDS, as opposed to emulsions or microemulsions, spontaneously form emulsions when added to an excess of water without any external mechanical dispersion or agitation. An advantage of SEDDS is that only gentle mixing is required to distribute the droplets throughout the solution. Additionally, water or the aqueous phase can be added just prior to administration, which ensures stability of an unstable or hydrophobic active ingredient. Thus, the SEDDS provides an effective delivery system for oral and parenteral delivery of hydrophobic active ingredients. SEDDS may provide improvements in the bioavailability of hydrophobic active ingredients. Methods of producing self-emulsifying dosage forms include, but are not limited to, for example, U.S. Pat. Nos. 5,858,401, 6,667,048, and 6,960,563.

Buccal formulations that include compounds described herein may be administered using a variety of formulations, which include, but are not limited to, U.S. Pat. Nos. 4,229,447, 4,596,795, 4,755,386, and 5,739,136. In addition, the buccal dosage forms described herein can further include a bioerodible (hydrolysable) polymeric carrier that also serves to adhere the dosage form to the buccal mucosa. The buccal dosage form is fabricated so as to erode gradually over a predetermined time period, wherein the delivery of the compound is provided essentially throughout. Buccal drug delivery avoids the disadvantages encountered with oral drug administration, e.g., slow absorption, degradation of the active agent by fluids present in the gastrointestinal tract and/or first-pass inactivation in the liver. With regard to the bioerodible (hydrolysable) polymeric carrier, virtually any such carrier can be used, so long as the desired drug release profile is not compromised, and the carrier is compatible with the compounds described herein, and any other components that may be present in the buccal dosage unit. Generally, the polymeric carrier comprises hydrophilic (water-soluble and water-swellable) polymers that adhere to the wet surface of the buccal mucosa. Examples of polymeric carriers useful herein include acrylic acid polymers and co, e.g., those known as "carbomers." Other components may also be incorporated into the buccal dosage forms described herein include, but are not limited to, disintegrants, diluents, binders, lubricants, flavoring, colorants, preservatives, and the like. For buccal or sublingual administration, the compositions may take the form of tablets, lozenges, or gels formulated in a conventional manner.

Transdermal formulations described herein (not claimed) may be administered using a variety of devices including but not limited to, U.S. Pat. Nos. 3,598,122, 3,598,123, 3,710,795, 3,731,683, 3,742,951, 3,814,097, 3,921,636, 3,972,995, 3,993,072, 3,993,073, 3,996,934, 4,031,894, 4,060,084, 4,069,307, 4,077,407, 4,201,211, 4,230,105, 4,292,299, 4,292,303, 5,336,168, 5,665,378, 5,837,280, 5,869,090, 6,923,983, 6,929,801 and 6,946,144. The transdermal dosage forms described herein may incorporate certain pharmaceutically acceptable excipients, which are conventional in the art. In one embodiment, the transdermal formulations described herein include at least three components: the combination of effective amounts of capsaicin, caffeine, and an alpha-agonist; (2) a penetration enhancer; and (3) an aqueous adjuvant. In addition, transdermal formulations can include additional components such as, but not limited to, gelling agents, creams and ointment bases, and the like. In some embodiments, the transdermal formulation can further include a woven or non-woven backing material to enhance absorption and prevent the removal of the transdermal formulation from the skin. In other embodiments, the transdermal formulations described herein can maintain a saturated or supersaturated state to promote diffusion into the skin.

In certain embodiments, delivery systems for the active agents (i.e., the capsaicin, caffeine, and an alpha-agonist) may be employed, such as, for example, liposomes and emulsions. In certain embodiments, compositions provided herein also include a mucoadhesive polymer, selected from among, for example, carboxymethylcellulose, carbomer (acrylic acid polymer), poly (methylmethacrylate), polyacrylamide, polycarbophil, acrylic acid/butyl acrylate copolymer, sodium alginate and dextran.

In some embodiments, the active agents described herein may be administered topically and are formulated into a variety of topically administrable compositions, such as solutions, suspensions, lotions, gels, pastes, medicated sticks, balms, creams or ointments. Such pharmaceutical compounds can contain solubilizers, stabilizers, tonicity enhancing agents, buffers and preservatives.

Generally, an active agent, such as capsaicin, caffeine, and an alpha-agonist, is administered in an amount effective for amelioration of, or prevention of the development of symptoms of, syncope (i.e., a therapeutically effective amount). Thus, a therapeutically effective amount can be an amount that is capable of at least partially preventing syncope or the onset of a syncope attack. The dose required to obtain an effective amount may vary depending on the agent, formulation, disease or disorder, and individual to whom the agent is administered.

The formulations and compositions described herein can be administered prior to, concurrently with and subsequent to the appearance of symptoms of a disease or disorder. In some embodiments, an agent is administered to a subject with a family history of syncope or who has a phenotype that may indicate a predisposition to syncope, or who has a genotype which predisposes the subject to syncope.

In some embodiments, the compositions described herein are provided as pharmaceutical and/or therapeutic compositions. The pharmaceutical and/or therapeutic compositions of the present invention can be administered in a number of ways depending upon whether local or systemic treatment is desired and upon the area to be treated.

According to the invention, administration is sublingual. Other non-claimed administration can be oral, buccal, nasal, transdermal or topical. Compositions and formulations can include transdermal patches, ointments, lotions, creams, gels, drops, suppositories, sprays, liquids and powders. Conventional carriers; aqueous, powder, or oily bases; thickeners; and the like can be necessary or desirable. Compositions and formulations for oral administration include powders or granules, suspensions or solutions in water or non-aqueous media, capsules, sachets or tablets. Thickeners, flavoring agents, diluents, emulsifiers, dispersing aids or binders can be desirable. Pharmaceutical and/or therapeutic compositions of the present invention include, but are not limited to, solutions, emulsions, and liposome containing formulations. These compositions can be generated from a variety of components that include, but are not limited to, preformed liquids, self-emulsifying solids and self-emulsifying semisolids.

In some embodiments, the active agents (e.g., capsaicin, caffeine and an alpha-agonist) are provided in a fluid that can be used for atmospheric treatment, such as by a mist, or delivered as a gel or foam. In some embodiments, the present invention provides a device comprising a reservoir, a pump, and a nozzle, wherein the reservoir comprises a fluid comprising the active agents that can be expelled via the pump through the nozzle to provide a mist, gel or foam comprising an effective dose of the active agents. In some embodiments, the active agents are provided as an aerosol spray in an appropriate aerosol spray-dispensing device. Accordingly, in some embodiments, the present invention provides a device or composition comprising the active agents and an aerosol propellant. Propellants include, but are not limited to, mixtures of volatile hydrocarbons, typically propane, n-butane and isobutene, dimethyl ether (DME), methyl ethyl ether, nitrous oxide, carbon dioxide and hydrofluoroalkanes (HFA): either HFA 134a (1,1,1,2,-tetrafluoroethane) or HFA 227 (1,1,1,2,3,3,3-heptafluoropropane) or combinations of the two. Typically, the active agents will be miscible with the propellant. The active agents may preferably be formulated to be dispensed as an aerosol mist, foaming gel, cream or lotion.

Modern aerosol spray products have three major parts; the can, the valve and the actuator or button. The can is most commonly lacquered tinplate (steel with a layer of tin) and may be made of 2 or 3 pieces of metal crimped together. Aluminum cans are also common and are generally used for more expensive products. The valve is crimped to the rig of the can, the design of this component is important in determining the spray rate. The actuator is depressed by the user to open the valve; the shape and size of the nozzle in the actuator controls the spread of the aerosol spray.

In some embodiments, the devices of the present invention comprise a piston barrier system. Packaging that uses a piston barrier system is often used for highly viscous products such as post-foaming gels, thick creams and lotions. The main benefit of the piston barrier system is that is assures separation of the product from the propellant, maintaining the purity and integrity of the formulation throughout its consumer lifespan. The piston barrier system also provides a controlled and uniform product discharge rate with minimal product retention and is economical.

In some embodiments, the devices of the present invention comprise a bag-in-can system (or BOV "bag on valve"). This system separates the product from the pressurizing agent with a hermetically-sealed, multi-layered laminated pouch, which maintains complete formulation integrity so only pure product is dispensed. In this embodiment, the active agent fluid is provided in the bag. Among its many benefits, the bag-in-can system extends a product's shelf life. Other advantages include all-attitude (360-degree) dispensing, quiet and non-chilling discharge.

The pharmaceutical formulations described herein may be in unit dosage forms suitable for single administration of precise dosages. In unit dosage form, the formulation is divided into unit doses containing appropriate quantities of one or more compound. The unit dosage may be in the form of a package, container or device containing discrete quantities of the formulation.

Dosing and administration regimens are tailored by the clinician, or others skilled in the pharmacological arts, based upon well-known pharmacological and therapeutic considerations including, but not limited to, the desired level of therapeutic effect, and the practical level of therapeutic effect obtainable.

### EXPERIMENTAL

### Example 1 (Reference example)

### Studies on the Effect of Capsaicin and Caffeine on Prevention of Syncope

**Patients and methods:** In a first study, we tested the effect of caffeine 200 mg plus capsaicin 50µL. The study was undertaken in 23 patients affected by VVS. They underwent head-up tilt (HUT) test as previously described (Brignole et al 2001). HUT was performed in a quiet room at 21°C. Patients were instructed to lie down on the tilt table for 15 min then tilted to 60° for 20-45 min. If no significant hemodynamic change occurred, the test was continued after sublingual administration of 300 mg of nitroglycerin (Natispray^{®}, Teofarma, It). Heart rate (HR) and systolic BP (SBP) were monitored continuously using 6-lead electrocardiogram and non-invasive beat-to-beat photopletismographic blood pressure monitoring. For the purpose of this study, the instant values of HR and SBP were taken at minutes 0.5, 1, 2, 3, 5, 10, 15 and 20 as measure of outcome.

The Caffeine plus Capsaicin mixture was administered at the time of onset of symptoms of impending syncope in 19 patients (12 women and 7 men, mean age 34±14 years) (Table 1). In one case, syncope was sudden without prodrome, and therefore the administration of the mixture could not be done. Caffeine plus capsaicin was also administered at the end of HUT in 3 patients who did not have VVS during the test.

In a second study, we tested the effect of Capsaicin alone (50µL Capsaicin 0.5%) on HR and BP in 3 healthy subjects (1 woman and 2 men mean age 35±18 years) and in 1 patient with a history of VVS during tilt table testing (Table 1).

**Drug Preparation:** 200 mg of Caffeine (SIGMA Aldrich^{®}) and 50µL of Capsaicin (0.5% (SIGMA Aldrich)) were dissolved in 1 mL of ethanol. Because at this concentration Caffeine is poorly soluble, the mixture was maintained at 40°C until administration.

**Drug administration:** The mixture was stored in a syringe and administered sublingual. Patients were instructed to roll the mixture under the tongue without spitting or swallowing it. They were also advised about the sharp and bitter character of the mixture. The administration of the drug was performed at the beginning of the prodromal period, when patients described sweat, hot flashes, or severe discomfort. In 5 cases we collected blood samples for caffeine dosage before, and at 30s, 1 min and 2 min after drug administration.

### Results

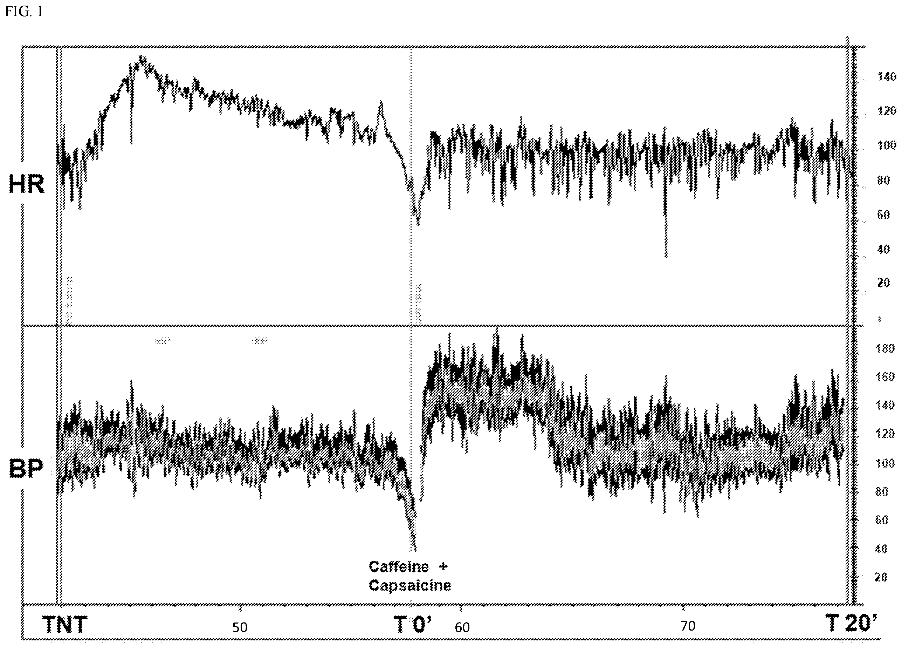

**Study 1:** In the 19 patients with prodromes, SBP decreased at least of 20mm Hg from baseline at the time of the mixture administration. We observed a drop in SBP from a mean of 135±18 mmHg before to a mean of 85±12 mmHg during prodromes. The administration of the mixture caused a rapid increase in SBP to a mean of 116±20 mmHg, which was associated with disappearance of symptoms in 15 cases (79%) (FIG. 1). The effect reached its maximum after a mean of 1.8±0.8 minutes and was present as early as 30 seconds after drug administration. The administration of the mixture failed to abort symptoms in 4 cases. One patient (male) was a big consumer of coffee (more than 600 mg/day). In the remaining 3 cases, we postulated that the drug was probably administered too late. A similar increase in SBP was observed in the 3 patients who received the drug mixture at the end of the HUT.

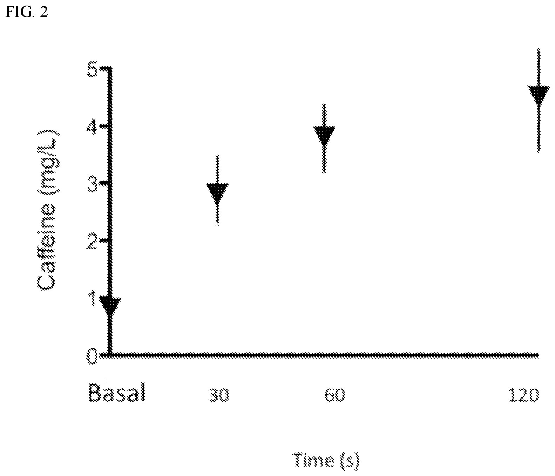

In 5 cases, blood was collected to measure Caffeine levels. We observed an increase in caffeine blood concentration starting at 30 seconds (see FIG. 2).

**Study 2:** Capsaicin alone increased SBP in 3 healthy subjects tested from a mean of 120±10 mmHg to 155±5 mmHg starting at 30 seconds after sublingual administration. In the patient with VVS, the administration of capsaicin alone at the onset of prodromes during tilt table testing resulted in an increase in SBP from 100 to 120mmHg. The patient experienced only presyncope (data not shown).

**Conclusion:** We found that among the 19 patients with a prodromal state, the sublingual
administration of the mixture of caffeine plus capsaicin was effective in increasing SBP, to abort symptoms and to prevent syncope occurrence in 79% of cases. The effects of caffeine and capsaicin were synergistic. Capsaicin alone was able to increase SBP in healthy subjects and prevent loss of consciousness in one VVS patient.

**Table 1. Tilt test results**

| **Pt n°** | **Age** | **Sex** | **Pending syncope during HUT** | **SBP at time 0 (drug admin), mmHg** | **Max SBP after drug** | **HR at time 0 (drug admin), bpm** | **HR after Drug** | **Time of max SBP increa se (min)** | **Symptoms after drug** |
|---|---|---|---|---|---|---|---|---|---|
| **Study 1 (VVS patients received Caffeine + Capsaicin during tilt testing)** | | | | | | | | | |
| 1 | 25 | F | YES | 65 | 155 | 67 | 111 | 1 | no |
| 2 | 39 | F | YES | 74 | 120 | 90 | 77 | 1 | no |
| 3 | 27 | F | YES | 97 | 110 | 95 | 80 | 2 | yes |
| 4 | 21 | F | YES | 80 | 95 | 90 | 100 | 2 | yes |
| 5 | 15 | F | YES | 105 | 145 | 112 | 76 | 3 | no |
| 6 | 55 | M | YES | 75 | 85 | 105 | 105 | 1 | yes |
| 7 | 18 | F | YES | 75 | 110 | 55 | 80 | 1 | no |
| 8 | 27 | F | YES | 85 | 125 | 60 | 90 | 2 | no |
| 9 | 43 | F | YES | 90 | 130 | 104 | 120 | 3 | no |
| 10 | 54 | F | YES | 80 | 100 | 98 | 115 | 1 | no |
| 11 | 60 | M | YES | 100 | 140 | 70 | 90 | 3 | no |
| 12 | 21 | F | YES | 90 | 120 | 104 | 118 | 1 | no |
| 13 | 54 | M | YES | 70 | 110 | 90 | 106 | 2 | no |
| 14 | 28 | F | YES | 75 | 90 | 48 | 78 | 1 | yes |
| 15 | 25 | F | YES | 90 | 125 | 100 | 76 | 2 | no |
| 16 | 24 | M | YES | 80 | 110 | 67 | 60 | 3 | no |
| 17 | 28 | M | YES | 100 | 130 | 110 | 80 | 2 | no |
| 18 | 45 | M | YES | 80 | 85 | 55 | 65 | 1 | yes |
| 19 | 33 | M | YES | 100 | 125 | 68 | 90 | 2 | no |
| | | | | | | | | | |
| **Mean** | **34** | | | **85** | **116** | **84** | **90** | **1.8** | |
| **±SD** | **14** | | | **12** | **20** | **21** | **18** | **0.8** | |
| 1 | 21 | F | NO | 120 | 120 | 145 | 145 | 10 | no |
| 2 | 61 | F | NO | 115 | 150 | 81 | 82 | 1 | no |
| 3 | 60 | M | NO | 124 | 136 | 78 | 82 | 1 | no |
| **Mean** | **47** | | | **129** | **135** | **101** | **103** | | |
| **±SD** | **23** | | | **4.5** | **15.0** | **37.8** | **36.4** | | |

| **Study 2 (Healthy subjects received Capsaicin alone during tilt testing)** | | | | | | | | | |
|---|---|---|---|---|---|---|---|---|---|
| 1 | 25 | F | NO | 120 | 160 | 80 | 100 | 1 | no |
| 2 | 56 | M | NO | 130 | 155 | 84 | 104 | 2 | no |
| 3 | 25 | M | NO | 110 | 150 | 70 | 85 | 1 | no |
| **Mean** | **35** | | | **120** | **155** | **78** | **96** | **1,3** | |
| **±SD** | **18** | | | **10** | **5** | **7** | **10** | **0,6** | |

| **(1 patient with VVS received Capsaicin alone during tilt testing)** | | | | | | | | | |
|---|---|---|---|---|---|---|---|---|---|
| 1 | 61 | H | YES | 100 | 120 | 56 | 80 | 1 | no |

### Example 2

### Safety and Pharmacokinetic Study in Normal Adult Volunteers of a Combination Oral Formula for Preventing Syncope

### Study Objectives and Endpoints

### Objectives

The objectives of this study were to determine the tolerability, safety, and pharmacokinetics of oral (sublingual) administration of capsaicin, phenylephrine and caffeine (CPC) mixture in normal, healthy adults.

**Primary Objective:** To characterize the tolerability and safety of a formulation of capsaicin, phenylephrine and caffeine (CPC) in normal adult volunteers.

**Secondary Objective(s):** 1) Characterize the pharmacokinetics (PK) of the CPC formulation in normal adult volunteers and 2) Determine the dose of phenylephrine (if any) needed to achieve a target increase in systolic BP of at least 40 mm Hg when combined with capsaicin and caffeine within 15 minutes of drug administration in 5 out of 6 subjects.

### Endpoints

**Primary Endpoint:**
   Tolerability: Number of subjects that complete both drug administrations
   Safety: Number of SAEs or systolic BP>190 mmHg
**Secondary Endpoint:**
   To characterize the pharmacokinetics (PK) of each of the drug active components
   To determine the dose of phenylephrine (if any) needed to achieve a target increase in systolic BP of at least 40 mm Hg when combined with capsaicin and caffeine within at least 15 minutes after drug administration in 5 out of 6 subjects.

### Drug Composition:

### Packaging and Labeling

### Active Components

| | |
|---|---|
| Capsaicin | 1 mg/mL |
| Caffeine | 200 mg/mL |
| Phenylephrine | 0, 600, 1200, or 1800 mcg/mL |

### Inactive Components

PVP
PEG 3350
Peppermint Oil
FD&C Blue #1 Lake

**Study Methods:** The design was an open label, phase I, dose escalation, pharmacokinetic study of a novel gel formulation of capsaicin, phenylephrine, and caffeine administered sublingually to normal, healthy adults.

**Dosing and Administration:** The product was expressed as fast as feasible from the oral syringe sublingually. The subject was asked to have the gel reside under the tongue for 1 minute before it was swallowed and the mouth was rinsed with water. Multiple rinses were allowed.

Of the three active components of the sublingual dosage, the dose of phenylephrine was the focus of dose escalation. The amount of caffeine and capsaicin in each dose were held constant at 200mg and 1mg, respectively. The amount of phenylephrine was increased in a modified Fibonacci escalation to identify the dose that both yielded an increase in systolic blood pressure (SBP) of at least 40 mmHg above baseline, yet did not exceed a SBP of 190 mmHg or greater.

**Table 2: Dose Levels**

| **Level** | **Phenylephrine** | **Caffeine** | **Capsaicin** |
|---|---|---|---|
| 0 | 0 mcg | 200 mg | 1 mg |
| 1 | 600 mcg | 200 mg | 1 mg |
| 2 | 1200 mcg | 200 mg | 1 mg |
| 3 | 1800 mcg | 200 mg | 1 mg |

The Maximally Tolerated Dose (MTD) of phenylephrine (PE) was defined as the PE dose that caused an increase in SBP above 190 mmHg in less than 33% of doses. This is based upon of the two-step (BD) model of the Phase I dose escalation schemes evaluated by Storer. This dose escalation scheme does not escalate dose within an individual. Instead, a single subject is exposed to the first dose level. If the subject does not experience a DLT, the next subject is exposed to the next, higher dose. The use of a single subject per dose level continues until the highest dose level of PE is reached (1800 mcg), or until a DLT is experienced. A DLT was defined as a CTCAE defined AE Grade 4, SAE, or SBP > 190 mmHg. If a subject developed a DLT after the first dose but before the second dose, the second dose was not given.

Once a single subject was successfully dosed at a phenylephrine dose of 1800 mcg OR experiences a DLT, additional 1-5 subjects were added to that dose level. De-escalation to the prior dose level occurred if and when two of these 2-6 subjects experience a DLT. If no additional subjects in this cohort of 6 subjects experienced a DLT, dose escalation was continued with 1 subject at the next higher dose level. The MTD was defined as the phenylephrine dose (in combination with 200mg caffeine and 1mg capsaicin) that caused an MTD in less than 33% of the subjects treated at that dose level (viz., 0-1 subjects with DLT in 6 subjects treated at the MTD).

### Dosing and PK Visit

There was one Dosing and PK visit for this study; the procedures performed at this visit are described below.

**Clinical Research Unit Admission:** Within 15 days of the in-person screening visit, the patient was admitted at the Clinical Research Unit (CRU), and standard admissions procedures occurred. A nurse obtained vital signs and placed a peripheral venous catheter. A urine sample was collected to confirm absence of pregnancy, if applicable. Negative pregnancy test results were confirmed prior to dosing.

For the overnight stay, subjects had a documented medical history and physical exam on admission and at discharge per UW Hospital admission requirements. In addition, assessment of time of last intake of caffeine, capsaicin or phenylephrine was obtained. The subjects were fasting within 2 hours of the drug dosing until 1 hour after second dose of CPC. For the remainder of the hospital stay, a caffeine and capsaicin restricted general diet was provided.

**Study Drug Administration:** The subject were asked to sit in a chair that allowed access to their mouth and subsequent blood sampling and continuous BP and HR monitoring. After 15-minute rest, vital signs were taken pre-dose, and SBP had to be ≤ 130 mmHg in order for dose to be given. If SBP > 130 mmHg, the blood pressure was checked again in 15 minutes. If the repeat measurement had SBP ≤ 130 mmHg, the first dose of CPC was given.

The study nurse delivered the appropriate dose to the subject using an oral syringe sublingually. The subject allowed the gel to reside under the tongue for 1 minute before the gel was swallowed and the mouth was rinsed with water that was swallowed. Multiple rinses were allowed as was water ad lib. Two hours later (following the 2-hour PK sample), the sublingual dosing was repeated, if SBP was ≤ 130 mmHg. If the second dose could not be given at the 2- hour time point, it was not given at all.

Oral discomfort or pain was measured using a 0-10 numeric pain scale (NPS), with 0 being no discomfort or pain, and 10 being pain as severe as can be imagined. The NPS was administered following the first and repeat dose, and prior to each PK blood collection.

**Pharmacokinetic Sampling:** Blood samples were collected via the venous catheter with appropriate pre- and post- sample flushing. If the indwelling venous catheter patency was lost, the phlebotomist or nurse attempted to replace it, if possible. Separate needle sticks could be done for the blood collection.

Samples were collected in 6 mL heparinized tubes at the following time points: Pre-dose, 30 seconds, 1, 2, 5, 15, 30, 45, 60, 90, 120 min, and 3, 4, 6, 8, 12, 24* hours
With the 24-hour sample* additional blood collection were done for CBC with Differential and Platelet Count, and Comprehensive Metabolic Panel.

At each PK time point, 1 - 4 ml collection tube with lithium heparin added for phenylephrine sample, and 2 - 2 mL collection tubes with K2EDTA for caffeine and capsaicin samples were obtained. The lithium heparin tube and 1 K2EDTA tube (capsaicin sample) were immediately placed on ice or refrigerated. The second K2EDTA tube (caffeine sample) remained at room temperature. All blood samples were centrifuged as soon as possible after clotting in a refrigerated centrifuge. The plasma from each tube was equally distributed between 2 polypropylene tubes, labeled and promptly stored at -80 °C until thawed for analysis.

**Results:** A total of 9 subjects received the drug with no serious side effects reported.

**Table 3: Pain score during the first 30 minutes following drug administration**

| **Subject** | **Ag** | **PE Dose (mg)** | **30 Seconds** | **1 Min** | **2 Min** | **5 Min** | **10 Min** | **15 Min** | **30 Min** |
|---|---|---|---|---|---|---|---|---|---|
| 103* | 19 | 0 | 8 | 10 | 8 | 6 | - | 1 | 1 |
| 104 | 22 | 60 | 6 | 5 | 6 | 3 | - | 1 | 0 |
| 113 | 33 | 120 | 2 | 2 | 3 | 3 | 0 | 0 | 0 |
| 106* | 26 | 180 | 2 | 5 | 7 | 5 | 2 | 0 | 0 |
| 107* | 26 | 180 | 1 | 3 | 5 | 1 | 0 | 0 | 0 |
| 109** | 27 | 180 | 3 | 6 | 7 | 2 | 1 | 0 | 0 |
| 110* | 30 | 180 | 2 | 4 | 5 | 3 | - | 1 | 0 |
| 111** | 35 | 180 | 4 | 8 | 9 | 5 | 0 | 0 | 0 |
| **Averag** | **27** | | **4** | **5** | **6** | **4** | **1** | **0** | **0** |
| **SD** | **5** | | **1.6** | **1.9** | **1.9** | **1.4** | **0.8** | 0.4 | **0.0** |

| | | | | | | | | | |
|---|---|---|---|---|---|---|---|---|---|
| * Gastric distress; ** Received milk or bread | | | | | | | | | |

**Table 4: Cuff systolic BP changes during the first 30 minutes after the first CPC dose administration**

| **Dose 1** | | | | | | | | | | |
|---|---|---|---|---|---|---|---|---|---|---|
| **Subject** | **Age** | **PE Dos e** | **Baselin e** | **1 Min** | **2 Min** | **5 Min** | **10 Min** | **15 Min** | **20 Min** | **30 Min** |
| 10 | 1 | 0 | 11 | 15 | 12 | 13 | 11 | 12 | 12 | 13 |
| 10 | 2 | 600 | 12 | - | 14 | 14 | 13 | 14 | 13 | 13 |
| 11 | 3 | 1200 | 12 | 14 | 15 | 14 | 14 | 14 | 15 | 14 |
| 10 | 2 | 1800 | 10 | - | 11 | 11 | 15 | 12 | 11 | 10 |
| 10 | 2 | 1800 | 11 | 12 | - | 13 | 14 | 12 | 12 | 12 |
| 10 | 2 | 1800 | 10 | 12 | 12 | 11 | 12 | 12 | 12 | 12 |
| 11 | 3 | 1800 | 12 | 14 | 14 | 14 | 14 | 14 | 14 | 13 |
| 11 | 3 | 1800 | 10 | - | 13 | 13 | 11 | 13 | 12 | 11 |
| **Averag** | **2** | | **11** | **13** | **13** | **13** | **13** | **13** | **13** | **12** |
| **SD** | **5** | | **8.4** | **10.6** | **15.5** | **10.2** | **14.7** | **10.5** | **11.9** | **11.3** |

**Table 5: Cuff systolic BP changes during the first 30 minutes after the second CPC dose administration**

| **Dose 2** | | | | | | | | | | |
|---|---|---|---|---|---|---|---|---|---|---|
| **Subject** | **Age** | **PE Dos e** | **Baselin e** | **1 Min** | **2 Min** | **5 Min** | **10 Min** | **15 Min** | **20 Min** | **30 Min** |
| 10 | 1 | 0 | 11 | - | 13 | - | 11 | 11 | - | 10 |
| 10 | 2 | 600 | 12 | - | 15 | 14 | 14 | 15 | 14 | 12 |
| 11 | 3 | 1200 | 11 | 14 | 13 | 13 | 13 | - | 12 | |
| 10 | 2 | 1800 | 10 | 10 | 11 | 12 | 11 | 11 | 11 | 10 |
| 107 | 2 | 1800 | 13 | - | - | - | - | - | - | - |
| 10 | 2 | 1800 | 12 | 14 | 13 | 12 | 10 | 11 | - | 11 |
| 11 | 3 | 1800 | 12 | 12 | 15 | 15 | 14 | 13 | 14 | 13 |
| 11 | 3 | 1800 | 11 | 11 | 14 | 14 | 14 | 12 | 13 | 11 |
| **Averag** | **2** | | **11** | **12** | **13** | **13** | **12** | **12** | **13** | **11** |
| **SD** | **5** | | **10.5** | **17.8** | **12.4** | **10.4** | **14.0** | **15.7** | **12.9** | **11.4** |

| | | | | | | | | | | |
|---|---|---|---|---|---|---|---|---|---|---|
| * Dose 2 was not given because baseline SBP was > 130 mmHg | | | | | | | | | | |

In summary, we saw a mean increase in systolic BP of around 20 mmHg starting at minute 1 that persisted for 10 minutes, followed by a gradual decline. SBP remained around 10 mmHg above baseline at 30 minutes following drug administration. No serious side effects were reported.

PE blood levels were consistently below 0.1mcg/L. A concentration of 9mcg/L has been shown to be associated with an increase in systolic BP of 6-20mmHg with a mean of 11mmHg (Eur J Clin Pharmacology (1986) 30:427-431). We made revisions to our protocol to increase the PE concentration in the CPC product to 10, 20 and 30 mg with no change in the escalation method. Continuation of the Phase 1 study with higher concentrations of PE was done and is now completed.

### Example 3

### Safety and Pharmacokinetic Study in Normal Adult Volunteers of a Combination Oral Formula for Preventing Syncope with Higher Levels of PE

This example provides additional data to Example 2. A total of 17 participants (including Example 2) received at least one dose of CPC. A total of 8 subjects received the higher doses of phenylephrine (PE)(10 mg (n=1), 20 mg (n=1), and 30 mg (n=6)). Combined with those who received lower doses of PE in 9 subjects (0 mcg (n=1), 600 mcg (n=1), 1200 mcg (n=1), and 1800 mcg (n=6)), a total of 17 subjects received the drug with no serious side effects reported.

### Subjects Information

The tables below are cumulative subject exposure to the CPC product based upon actual exposure data from beginning of enrollment until closure of enrollment.

**Table 6. Cumulative Subject Exposure**

| Treatment | Number of Subjects |
|---|---|
| Drug | 17 |
| Comparator | NA |
| Placebo | NA |

**Table 7. Cumulative Subject Exposure by Age and Sex**

| | Number of Subjects | | |
|---|---|---|---|
| Age Range | Male | Female | Total |
| 18 to 20 years | 0 | 1 | 1 |
| 20 to 30 years | 4 | 3 | 7 |
| 30 to 40 years | 3 | 6 | 9 |
| 40 to 50 years | 0 | 0 | 0 |

**Table 8. Cumulative Subject Exposure by Racial Group**

| Racial Group | Number of Subject |
|---|---|
| Asian | 3 |
| Black | 0 |
| Caucasian | 12 |
| Other | 1 (American Indian) |
| Unknown | 1 |
| Total | 17 |

### Study Results

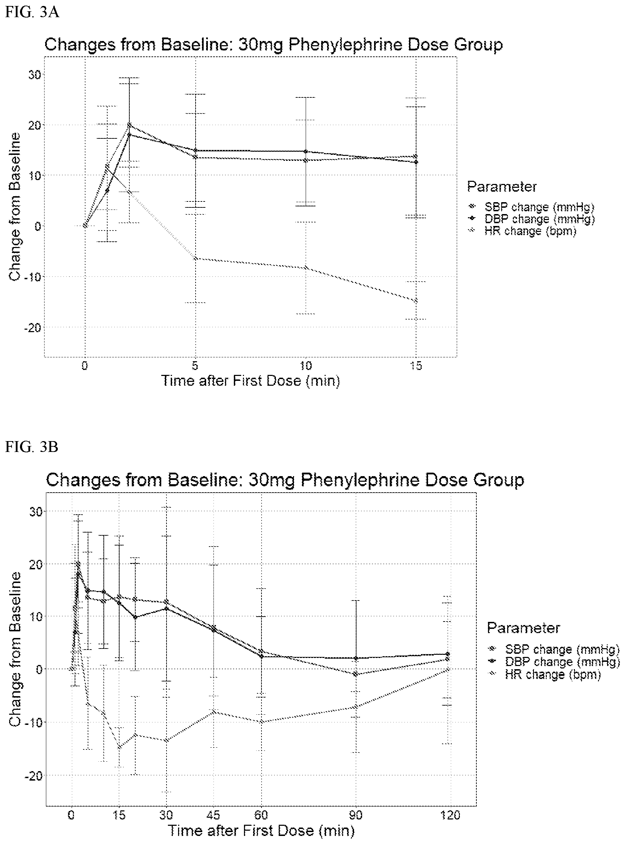

*Blood Pressure:* In the 6 subjects who received CPC with the highest dose of PE (Capsaicin 1 mg, Phenylephrine 30 mg, Caffeine 200 mg), the peak increase in SBP was at 2 minutes with an average of 21 mmHg (range 15-33 mmHg). The increase in BP was statistically significant for at least 15 minutes with a gradual decline over 2 hours. There was an initial increase in HR followed by a decrease to below baseline over the 2-hour period (Figures 3A and 3B).

*Pain Score:* The Pain Score (scale of 1-10) was highest at 2 minutes (mean 5, range 3-8) with a gradual decline to 1 (range 0-3) at 15 minutes. The mean was 3, 2 and 1 at 5 minutes, 10 minutes and 15 minutes, respectively. The Pain Score was 0 at 30 minutes in all subjects except for one who had a score of 1.

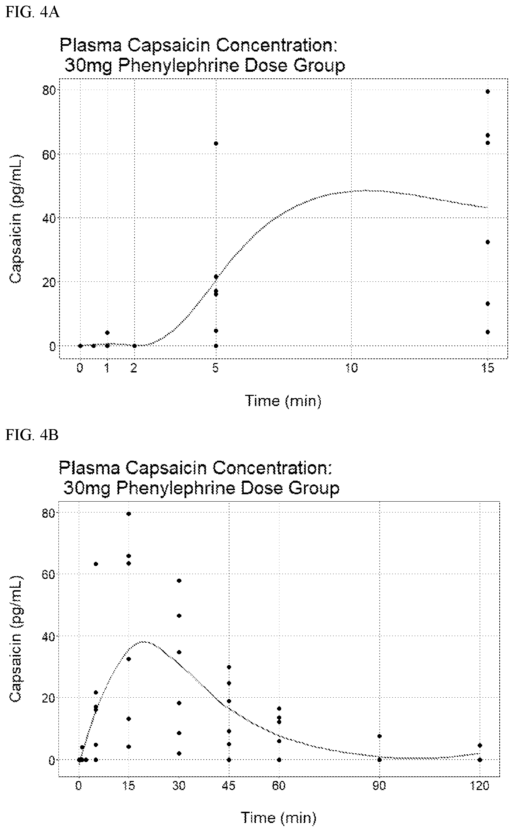

*Capsaicin blood levels:* Capsaicin blood levels ranged from < 4.00 pg/mL to 79.5 pg/mL. We saw a gradual increase in Capsaicin blood levels reaching a peak at around 15 minutes with a decline over the remaining 95 minutes (Figures 4A and 4B).

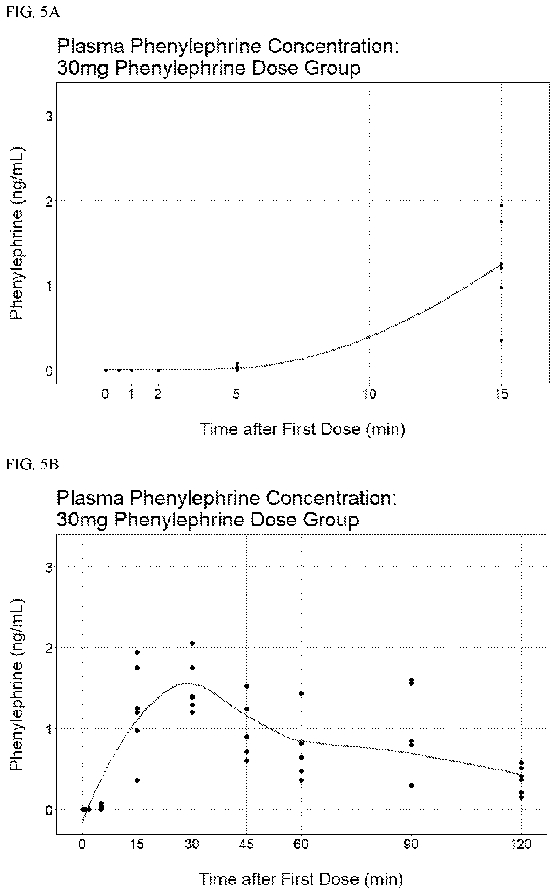

*Phenylephrine blood levels:* PE blood levels were undetectable in the first 15 minutes with an average around 1 ng/ml at 15 minutes, peaking to 1-2 ng/ml at 30 minutes with a gradual decline over 2 hours (Figures 5A and 5B).

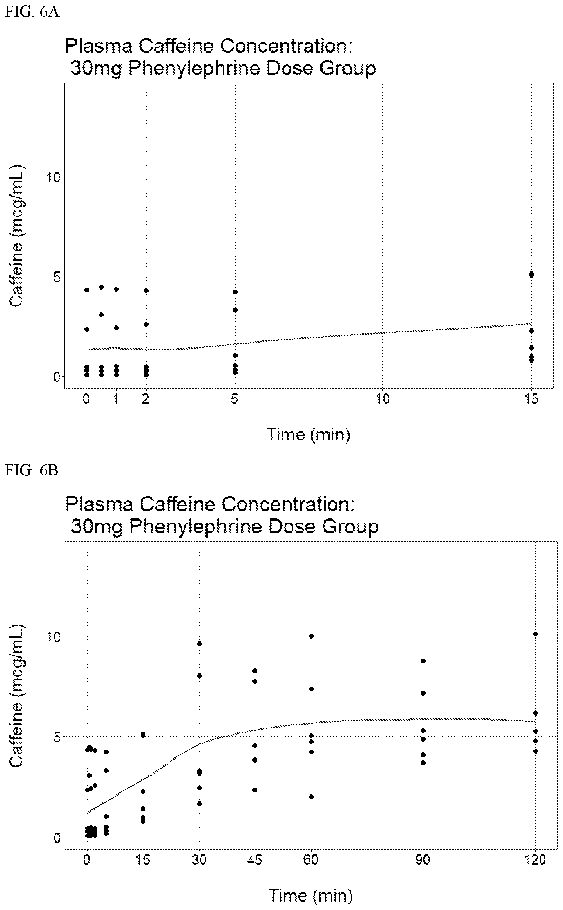

*Caffeine blood levels:* Caffeine blood levels were present in some subjects at baseline reflecting dietary caffeine intake. The blood levels were unchanged in the first 15 minutes (around 2-3 mcg/mL) suggesting the absence of sublingual absorption. There was a gradual increase after 15 minutes to 5-6 mcg/mL consistent with GI absorption. This blood level persisted for at least another 60 minutes i.e. end of the 2-hour period (Figures 6A and 6B).

### Adverse Events

In the Phase 1 study, the following non-serious adverse events were noted in the 17 volunteers who received the CPC drug. Of note, some subjects had more than one non-serious adverse event. All of these events were considered unexpected per the Phase 1 Protocol.

**Table 9. Non-Serious Adverse Events in Phase 1 PK Study**

| **System of Care*** | **Preferred Term*** | **Number of Subjects** |
|---|---|---|
| Respiratory Thoracic and Mediastinal Disorders | Throat Tightness | 1 |
| Gastrointestinal Disorders | Nausea | 2 |
| Metabolism and nutrition disorders | Hypoglycemia | 1 |
| Nervous System Disorders | Dizziness | 1 |
| Nervous System Disorders | Headache | 1 |
| Nervous System Disorders | Presyncope | 2 |
| Gastrointestinal Disorders** | Dyspepsia** | 6 |

Overall, we have demonstrated that the CPC product is safe in normal volunteers with no history of cardiovascular disease and ages 18-50 years old. The common side effects were oral and gastric discomfort, which were most likely related to Capsaicin.

The oral discomfort is a desired effect for the drug to work, as we believe it to be a trigger for increased sympathetic activity. Of note, none of the 17 volunteers, refused to hold CPC sub-lingually for 1 minute, and/or to swallow CPC or take the second dose of CPC. Refusal to do any of these 3 things was used as a marker of intolerance related to oral or gastric discomfort. Furthermore, in the 6 volunteers who received the highest dose of CPC, the oral Pain Score (scale of 1-10) was highest at 2 minutes (mean 5, range 3-8) with a gradual decline to 1 (range 0-3) at 15 minutes. The mean was 3, 2 and 1 at 5 minutes, 10 minutes and 15 minutes, respectively. The oral Pain Score was 0 at 30 minutes in all volunteers except for one who had a score of 1.

There was one vasovagal event that was thought to be the result of the frequent blood draws. A second volunteer had a vasovagal like reaction, with bleeding related to the loss of IV before being dosed with CPC. This is a known potential risk with any medical procedure including blood draws and IV placement.

### Study Interpretation

The results of our Phase 1 study suggest the CPC drug is safe. We attribute the early increase in BP (0-15 minutes) to Capsaicin and the late increase (15-120 minutes) to PE and Caffeine. We make this statement because both PE and Caffeine were barely detected in the blood during the first 15 minutes leaving only Capsaicin as the cause of early increase in BP. Conversely, the late increase in BP could only be attributed to PE and Caffeine because Capsaicin blood levels were too low to cause a systemic effect and the oral discomfort due to Capsaicin nearly subsided at 15 minutes (Mean Pain Score 1 at 15 minutes). Of note, the increases in BP 0-15 minutes after drug administration was similar with the lower doses of PE (n=9) when compared with the higher doses (n=8), suggesting once again that the early changes were due to Capsaicin.

*Capsaicin:* The molecular weight of capsaicin is 305g/mole. Accordingly, the highest detected blood level in our study of 79pg/mL or 79ng/L corresponds to 0.26pmole/L. The EC50 value for capsaicin on its VR1receptor is in the micro molar range (10⁻⁶ mole/L) (Caterina MJ et 1997 Nature 389: 816-824; Yang F et al 2015 Nat Chem Biol 11: 518-524.). Therefore, the ratio of the highest blood level detected to EC50 is 0.26 * 10⁻¹²/ 10⁻⁶. Simply stated, the capsaicin blood levels detected in this study were too low to cause any effect through the systemic circulation. We hypothesize the effects were due to local irritation and reflex increase in sympathetic activity. Indeed, the peak increase in SBP coincided with the highest Pain Score at 2 minutes and the decline in SBP at 15 minutes coincided with a mean Pain Score of 1 consistent with resolution of the oral discomfort.

*Phenylephrine:* A PE blood concentration of 20nM corresponding to 3mcg/L or 3ng/ml is associated with a mean increase of 3 mmHg in SBP (Eur J Clin Pharmacology (1986) 30:427-431). In the current study, the blood concentration was 1-2ng/ml. We speculate that the contribution of PE to SBP elevation was around 1-2 mmHg. Furthermore, we believe the effect was limited to the time period of 15-120 minutes when the blood levels started to exceed 1 ng/L.

*Caffeine:* A single dose of 200-250mg of Caffeine is associated with an increase in SBP of 3-14 mmHg within 30 minutes with a maximal increase at 60-120 minutes (Nurminen ML et al Coffee, caffeine and blood pressure: a critical review. Eur J Clin Nutrition 1999; 53: 831-839; Table 2). At a concentration of 3.7 mg/L, SBP increased from 132±12 to 133±10 mmHg (p<0.05) at 18 minutes post caffeine ingestion (3mg/Kg) (Renda J et al Genetic determinants of blood pressure responses to caffeine drinking. Am J Clin Nutrition 2012 ; 95: 241-248; Figure 2). In the current study, the caffeine dose was 200mg and the blood levels were 2-6mcg/ml or mg/L. We speculate that the contribution of Caffeine to SBP elevation was around 1-2 mmHg. Similar to PE, we believe the effect was limited to the time period of 15-120 minutes when the blood levels started to exceed 2 mg/L.

Despite the absence of significant blood levels of PE and Caffeine in the first 15 minutes post drug administration, we believe all 3 drug components should continue to be part of the product for the following reasons:
*1. Syncope prevention in high-risk situations:* We believe the CPC product has a role in not only aborting a pending vasovagal event but also in preventing one in high-risk situations. While Capsaicin plays a major role in the immediate effects (0-15 minutes), we believe PE and Caffeine play a significant role 15-120 minutes post-drug intake. For PE, the effects are directly linked to its vasopressor properties. For caffeine, the effects are due to its direct effects on BP and its role in blocking adenosine receptors.
   With caffeine, the effects on BP are likely to be minimal in the absence of syncope; however, we hypothesize that its effects are greater at the time of pending syncope due to the endogenous release of adenosine (Circulation 2002 Jul;106(5):569-74; Heart 2012 Jun;98(11):855-9.) and caffeine's ability to block adenosine receptors. Indeed, the caffeine blood levels after 15 minutes are high enough to counteract the cardiovascular effects of endogenous adenosine release at the time of vasovagal syncope. The affinity of caffeine for A1 and A2 adenosine receptors are 10 and 9 µM respectively (Jacobson KA, Adenosine A2A receptors antagonists: from caffeine to selective non-xanthines. Br J Pharmacol. 2020 May 18. doi: 10.1111/bph. 15103), corresponding to 1.9mg/L. The observed blood levels in our study at 15 minutes were certainly at least twice that amount.
*2. Effects of PE and Caffeine on fatigue after a syncopal event:* Up to 94% of patients experience fatigue following vasovagal syncope (Am Journal of Med 1995 Apr:98(4):365-73, Table 1), with some stating "I slept for hours as if I had run a marathon". We believe that PE and Caffeine will counteract these symptoms.

### Conclusion

Overall, CPC has been well tolerated in healthy adults, with no serious adverse reaction (SAR) to report. In the Phase 1 study, the maximum tolerated dose (MTD) of CPC was Capsaicin 1 mg, Phenylephrine 30 mg, Caffeine 200 mg.

### Example 4 (Prophetic)

### The Effect of Capsaicin-Phenylephrine-Caffeine Formulation on Aborting Tilt Induced Syncope in Patients with a History of Vasovagal Syncope

**Study protocol:** Double-blinded, acute, proof-of-efficacy, randomized study. Eligible patients will undergo tilt table testing (20 min passive + 15 min NTG phase) with continuous BP, HR and ECG monitoring. Patients will be randomized to receive CPC (1 mg Capsaicin, 30 mg Phenylephrine, 200 mg Caffeine) or placebo preparation to be administered at the onset of prodromal symptoms (blurred vision, lightheadedness, dizziness, epigastric discomfort, nausea, sweating, etc.).

### End-points

*Primary endpoint:* Comparison of the percentage of patients who have hypotensive syncope or near syncope with SBP ≤ 70 mmHg during tilt test in the active and placebo arms
Hypotensive syncope is defined as transient loss of consciousness (unresponsive to verbal commands) associated with a SBP ≤ 90 mmHg. Near syncope is defined as sensation of "near fainting" but still responsive to verbal commands. Near syncope will be used as a primary endpoint only when it is associated with a SBP ≤ 70 mmHg.

### Secondary endpoints:

- Time to syncope or near syncope after drug or placebo administration
- Percentage of patients who have asystolic pauses >3 sec in the CPC and placebo arms
- Measures of fatigue (Ordinal Scale 1-5) at 1, 4 and 8 hours after tilt test termination

**Sample size:** Knowing that the efficacy of current therapies for VVS range between 30% and 50%, we hypothesize a 50% reduction in syncope or near syncope rate with the CPC product. Thus in the intent to treat population, we assume that the active treatment arm will decrease the syncope rate from an absolute value of 62% to 34.1% (31% diluted with patients not completing the protocol, see statistical methods). A total of 126 patients (63 active and 63 placebo) are needed in order to achieve a statistical power of 85.2% to detect this difference using a 2-sided overall significance level of 0.05 with a Pocock boundary (i.e. z = ± 2.1599 at both interim and final analysis). Accounting for the number of subjects that might not meet eligibility criteria after virtual enrollment, inflating the total number of subjects to be enrolled to 132 patients should result in an adequate sample size. An interim analysis is predefined at the time in which 76 total patients are randomized.

It is expected that a single administration of sublingual CPC preparation during the prodromal phase aborts tilt-induced syncope or near syncope with SBP ≤ 70 mmHg in patients with a history of vasovagal syncope.

### References:

Ammirati F, Colivicchi F, Santini M. Effects of intravenous etilefrine in neurocardiogenic syncope induced by head-up tilt testing. Am J Cardiol. 2000 Aug 15; 86(4):472-4.

Arsyad A, Dobson GP. Adenosine relaxation in isolated rat aortic rings and possible roles of smooth muscle Kv channels, KATP channels and A2a receptors. BMC Pharmacol. Toxicol. 2016;17:23.

Brignole M, Alboni P, Benditt D, Bergfeldt L, Blanc JJ, Bloch Thomsen PE, van Dijk JG, Fitzpatrick A, Hohnloser S, Janousek J, Kapoor W, Kenny RA, Kulakowski P, Moya A, Raviele A, Sutton R, Theodorakis G, Wieling W; Task Force on Syncope, European Society of Cardiology. Guidelines on management (diagnosis and treatment) of syncope. Eur Heart J. 2001 Aug 22(15):1256-306.

Brignole M, Deharo JC, De Roy L, Menozzi C, Blommaert D, Dabiri L, Ruf J, Guieu R. Syncope due to idiopathic paroxysmal atrioventricular block: long-term follow-up of a distinct form of atrioventricular block. J Am Coll Cardiol. 2011 Jan 5; 58(2):167-73.

Brignole M, Solari D, Iori M, Bottom N, Guieu R, Deharo JC. Efficacy of theophylline in patients affected by low adenosine syncope. Heart Rhythm. 2016 May; 13(5):1151-4.

Cook WH, Convertino VA. Association between vasovagal hypotension and low sympathetic neural activity during presyncope. Clin Auton Res 2002 Dec12(6):483-6.

Day SC, Cook EF, Funkenstein H, Goldman L. Evaluation and outcome of emergency room patients with transient loss of consciousness. Am J Med 1982 Jul; 73(1):15-23.

Deharo JC, Mechulan A, Giorgi R, Franceschi F, Prevot S, Peyrouse E, Condo J, By Y, Ruf J, Brignole M, Guieu R. Adenosine plasma level and A2A adenosine receptor expression: correlation with laboratory tests in patients with neurally mediated syncope. Heart. 2012 Jun;98(11):855-9.

Deharo JC, Guieu R, Mechulan A, Peyrouse E, Kipson N, Ruf J, Gerolami V, Devoto G, Marre V, Brignole M. Syncope without prodromes in patients with normal heart and normal electrocardiogram: a distinct entity. J Am Coll Cardiol. 2013 Sep 17; 62(12):1075-80.

Fu Q, Levine BD. Pathophysiology of neutrally mediated syncope: Role of cardiac output and total peripheral resistance. Auton Neurosci 2014 Sep; 184: 24-26.

Ganzeboom KS, Mairuhu G, Reitsma JB, Linzer M, Wieling W, van Dijk N. Lifetime cumulative incidence of syncope in the general population: a study of 549 Dutch subjects aged 35-60 years. J Cardiovasc Electrophysiol 2006 Nov; 17: 1172-76.

Gelotte, CK, Zimmerman BA. Pharmacokinetics, safety, and cardiovascular tolerability of phenylephrine HCl 10, 20, and 30 mg after a single oral administration in healthy volunteers. Clin Drug Investig 2015; 35: 547-58.

Goyal P, Maurer MS. Syncope in older adults. J Geriatr Cardiol. 2016 ;13:380-6.

Guieu R, Deharo JC, Ruf J, Mottola G, Kipson N, Bruzzese L, Gerolami V, Franceschi F, Ungar A, Tomaino M, Lori M, Brignole M. Adenosine and Clinical Forms of Neurally-Mediated Syncope. J Am Coll Cardiol. 2015 Jul 14; 66(2):204-5.

Iwamoto T, Umemura S, Toya Y, Uchibori, T, Kogi K, Takagi N, Ishii M. Identification of adenosine A2 receptor-cAMP system in human aortic endothelial cells. Biochem. Biophys. Res. Commun. 1994; 199, 905-910.

Jardine DL, Melton IC, Crozier IG, English S, Bennett SI, Frampton CM, Ikram H. Decrease in cardiac output and muscle sympathetic activity during vasovagal syncope. Am J Physiol Heart Circ Physiol 2002 May; 282(5): H1804-9.

Kleppisch, T. ; Nelson, M.T. Adenosine activates ATP-sensitive potassium channels in arterial myocytes via A2 receptors and cAMP-dependent protein kinase. Proc. Natl. Acad. Sci U S A 1995;92, 12441-12445.

Lamb LE, Green HC, Combs JJ, Cheeseman SA, Hammond J. Incidence of loss of consciousness in 1,980 Air Force personnel. Aerosp Med 1960; 31: 973-988.

Morichetti A, Astorino G. [Epidemiological and clinical findings in 697 syncope events]. Minerva Med 1998 Jun; 89: 211-220.

Morillo CA, Eckberg DL, Ellenbogen KA, Beightol LA, Hoag JB, Tahvanainen KU, Kuusela TA, Diedrich AM. Vagal and sympathetic mechanisms in patients with orthostatic vasovagal syncope. Circulation 1997 Oct 21; 96(8): 2509-13.

Mosqueda-Garcia R, Furlan R, Tank J, Fernandez-Violante R. The elusive pathophysiology of neutrally mediated syncope. Circulation 2000 Dec 5;102: 2898-906.

Nordin M, Fagius J. Effect of noxious stimulation on sympathetic vasoconstrictor outflow to human muscles. J Physiol 1995 Dec 15; 489 (Pt 3): 885-894.

Olde Nordkamp LR, van Dijk N, Ganzeboom KS, Reitsma JB, Luitse JS, Dekker LR, Shen WI, Wieling W. Syncope prevalence in the ED compared to general practice and population: a strong selection process. Am J Emerg Med 2009 Mar; 27(3): 271-279.

Ponnoth DS, Sanjani MS, Ledent C, Roush K, Krahn T, Mustafa SJ. Absence of adenosine-mediated aortic relaxation in A(2A) adenosine receptor knockout mice. Am J. Physiol. Heart Circ. Physiol. 2009;297, H1655-H1660.

Raviele A, Giada F, Gasparini G. Efficacy of a patient-activated pharmacologic pump using phenylephrine as active drug and prodromal symptoms as a marker of imminent loss of consciousness to abort tilt-induced syncope. J Am Coll Cardiol. 2005 Jan 18; 45(2):320-1.

Riksen NP, Smits P, Rongen GA. The cardiovascular effects of methylxanthines. Handb Exp Pharmacol. 2011; (200): 413-37. Review.

Saadjian AY, Lévy S, Franceschi F, Zouher I, Paganelli F, Guieu RP. Role of endogenous adenosine as a modulator of syncope induced during tilt testing. Circulation. 2002 Jul 30;106(5):569-74. doi: 10.1161/01.cir.0000023924.66889.4c.

Saadjian AY, Gerolami V, Giorgi R, Mercier L, Berge-Lefranc JL, Paganelli F, Ibrahim Z, By Y, Guéant JL, Lévy S, Guieu RP. Head-up tilt induced syncope and adenosine A2A receptor gene polymorphism. Eur Heart J. 2009 Jun; 30(12):1510-5.

Serletis A, Rose S, Sheldon AG, Sheldon RS. Vasovagal syncope in medical students and their first-degree relatives. Eur Heart J 2006 Aug; 27(16): 1965-70.

Sheldon RS, Grubb BP 2nd, Olshansky B, Shen WK, Calkins H, Brignole M, Raj SR, Krahn AD, Morillo CA, Stewart JM, Sutton R, Sandroni P, Friday KJ, Hachul DT, Cohen MI, Lau DH, Mayuga KA, Moak JP, Sandhu RK, Kanjwal K. 2015 heart rhythm society expert consensus statement on the diagnosis and treatment of postural tachycardia syndrome, inappropriate sinus tachycardia, and vasovagal syncope. Heart Rhythm. 2015 Jun;12(6): e41-63.

Shen WK, Sheldon RS, Benditt DG, Cohen MI, Forman DE, Goldberger ZD, Grubb BP, Hamdan MH, Krahn AD, Link MS, Olshansky B, Raj SR, Sanduhu RK, Sorajja D, Sun BC, Yancy CW. 2017 ACC/AHA/HRS Guideline for the Evaluation and Management of Patients With Syncope: Executive Summary: A Report of the American College of Cardiology/American Heart Association Task Force on Clinical Practice Guidelines and the Heart Rhythm Society. J Am Coll Cardiol. 2017 Aug 1; 70(5):620-663.

Shryock JC, Belardinelli L. Adenosine and adenosine receptors in the cardiovascular system: biochemistry, physiology, and pharmacology. Am J Cardiol. 1997 Jun 19; 79(12A):2-10. Smutzer G, Devassy RK. Integrating TRPV1 Receptor Function with Capsaicin Psychophysics. Adv Pharmacol Sci. 2016:1512457.

Storer BE. Design and analysis of phase I clinical trials. Biometrics. 1989 Sep; 45(3): 925-37. Sumner GL, Rose MS, Koshman ML, Ritchie D, Sheldon RS, Prevention of Syncope Trial Investigators. Recent history of vasovagal syncope in a young, referral-based population is a stronger predictor of recurrent syncope than lifetime syncope burden. J Cardiovasc Electrophysiol 2010 Dec; 21(12):1375-80.

Sun BC. Quality-of-life, health service use, and costs associated with syncope. Prog Cardiovasc Dis 2013 Jan-Feb; 55(4): 370-5.

## Claims

1. A composition for use in treating or preventing a disease or condition selected from the group consisting of syncope, vasovagal reaction in blood donors, dialysis-induced hypotension, and symptomatic orthostatic hypotension, by sublingual administration, the composition comprising:
an effective amount per dose of capsaicin; and
an effective amount per dose of caffeine;
wherein the combined effective amounts of the capsaicin and caffeine are effective to prevent syncope, vasovagal reaction in blood donors, dialysis-induced hypotension, or symptomatic orthostatic hypotension when administered sublingually to a subject in need thereof;
wherein the composition further comprises an effective amount per dose of an α-agonist, preferably wherein the α-agonist is selected from the group consisting of phenylephrine and etilefrine (ethylphenylephrine), and preferably wherein the effective amount per dose of the α-agonist is from 1 to 100 mg, more preferably from 2 to 70 mg, even more preferably from 5 to 50 mg.

2. The composition for use of claim 1, wherein the effective amount per dose of capsaicin is from 0.1 to 10 mg, preferably 0.3 to 5 mg, more preferably 0.5 to 1.5 mg.

3. The composition for use of any one of claims 1 - 2, wherein the effective amount per dose of caffeine is from 50 to 400 mg, preferably 100 to 300 mg, more preferably 150 to 250 mg.

4. The composition for use of any one of claims 1 to 3, further comprising one of more pharmaceutically acceptable carriers, wherein the pharmaceutically acceptable carrier is compatible with sublingual administration.

5. The composition for use of claim 4, wherein the pharmaceutically acceptable carrier is selected from the group consisting of an aqueous carrier, a gelling agent and combinations thereof.

6. The composition for use of claim 5, wherein the aqueous carrier is selected from the group consisting of water, an alcohol, and combinations thereof.

7. The composition for use of claim 5, wherein the gelling agent is selected from the group consisting of polyvinylpyrrolidone, polyethylene glycol and combinations thereof.

8. The composition for use of claim 7, wherein the polyethylene glycol is selected from the group consisting of polyethylene glycol 3350, polyethylene glycol 400, and combinations thereof.

9. The composition for use of any one of claims 1 to 8, wherein the composition is an aqueous solution.

10. The composition for use of any one of claims 1 to 9, wherein the composition is an aqueous gel.

11. The composition for use of any one of claims 1 to 10, wherein the composition further comprises a flavoring agent and/or a stabilizing agent, and/or a coloring agent.

12. A sublingual drug delivery formulation for use in treating or preventing a disease or condition selected from the group consisting of syncope, vasovagal reaction in blood donors, dialysis-induced hypotension, and symptomatic orthostatic hypotension comprising:
the composition for use in accordance to any one of the previous claims;
one or more pharmaceutically acceptable carriers compatible with sublingual administration.

13. A drug delivery device comprising a reservoir containing one or more effective doses of the composition for use according to any one of claims 1 to 11 or formulation for use according to claim 12.

14. The drug delivery device of claim 13, wherein the device is activatable by a user and calibrated to deliver an effective dose of the composition for use according to any one of claims 1 to 11 or formulation for use according to claim 12 upon activation by a user.

## Patentansprüche

1. Zusammensetzung zur Behandlung oder Prävention einer Krankheit oder eines Leidens ausgewählt aus der Gruppe bestehend aus Synkopen, vasovagalen Reaktionen bei Blutspendern, dialyseinduzierter Hypotonie und symptomatischer orthostatischer Hypotonie, durch sublinguale Verabreichung, wobei die Zusammensetzung Folgendes umfasst:
pro Dosis eine wirksame Menge an Capsaicin; und
pro Dosis eine wirksame Menge an Koffein,
wobei die kombinierten wirksamen Mengen von Capsaicin und Koffein Synkopen, vasovagale Reaktionen bei Blutspendern, dialyseinduzierte Hypotonie oder symptomatische orthostatische Hypotonie verhindern, wenn sie sublingual an ein dessen bedürftiges Subjekt verabreicht werden,
wobei die Zusammensetzung weiterhin pro Dosis eine wirksame Menge eines α-Agonisten umfasst, wobei der α-Agonist vorzugsweise aus der aus Phenylephrin und Etilefrin (Ethylphenylephrin) bestehenden Gruppe ausgewählt ist und wobei die wirksame Menge des α-Agonisten pro Dosis vorzugsweise 1 bis 100 mg, besonders bevorzugt 2 bis 70 mg, noch mehr bevorzugt 5 bis 50 mg beträgt.

2. Zusammensetzung zur Verwendung nach Anspruch 1, wobei die wirksame Menge an Capsaicin pro Dosis 0,1 bis 10 mg, vorzugsweise 0,3 bis 5 mg, besonders bevorzugt 0,5 bis 1,5 mg beträgt.

3. Zusammensetzung zur Verwendung nach einem der Ansprüche 1 - 2, wobei die wirksame Menge an Coffein pro Dosis 50 bis 400 mg, vorzugsweise 100 bis 300 mg, besonders bevorzugt 150 bis 250 mg beträgt.

4. Zusammensetzung zur Verwendung nach einem der Ansprüche 1 bis 3, weiterhin umfassend einen oder mehrere pharmazeutisch unbedenkliche Trägern, wobei der pharmazeutisch unbedenkliche Träger mit sublingualer Verabreichung kompatibel ist.

5. Zusammensetzung zur Verwendung nach Anspruch 4, wobei der pharmazeutisch unbedenkliche Träger aus der aus einem wässrigen Träger, einem Geliermittel und Kombinationen davon bestehenden Gruppe ausgewählt ist.

6. Zusammensetzung zur Verwendung nach Anspruch 5, wobei der wässrige Träger aus der aus Wasser, einem Alkohol und Kombinationen davon bestehenden Gruppe ausgewählt ist.

7. Zusammensetzung zur Verwendung nach Anspruch 5, wobei das Geliermittel aus der aus Polyvinylpyrrolidon, Polyethylenglykol und Kombinationen davon bestehenden Gruppe ausgewählt ist.

8. Zusammensetzung zur Verwendung nach Anspruch 7, wobei das Polyethylenglykol aus der aus Polyethylenglykol 3350, Polyethylenglykol 400 und Kombinationen davon bestehenden Gruppe ausgewählt ist.

9. Zusammensetzung zur Verwendung nach einem der Ansprüche 1 bis 8, wobei die Zusammensetzung eine wässrige Lösung ist.

10. Zusammensetzung zur Verwendung nach einem der Ansprüche 1 bis 9, wobei die Zusammensetzung ein wässriges Gel ist.

11. Zusammensetzung zur Verwendung nach einem der Ansprüche 1 bis 10, wobei die Zusammensetzung weiterhin einen Geschmacksstoff und/oder ein Stabilisierungsmittel und/oder ein Farbmittel umfasst.

12. Sublinguale Formulierung zur Verabreichung von Arzneimitteln zur Verwendung bei der Behandlung oder Prävention einer Krankheit oder eines Leidens ausgewählt aus der Gruppe bestehend aus Synkopen, vasovagalen Reaktionen bei Blutspendern, dialyseinduzierter Hypotonie und symptomatischer orthostatischer Hypotonie, umfassend:
die Zusammensetzung zur Verwendung nach einem der vorhergehenden Ansprüche;
einen oder mehrere pharmazeutisch unbedenkliche Träger, die mit sublingualer Verabreichung kompatibel sind.

13. Arzneimittelabgabevorrichtung, umfassend ein Reservoir, das eine oder mehrere wirksame Dosen der Zusammensetzung zur Verwendung nach einem der Ansprüche 1 bis 11 oder der Formulierung zur Verwendung nach Anspruch 12 enthält.

14. Arzneimittelabgabevorrichtung nach Anspruch 13, wobei die Vorrichtung von einem Anwender aktivierbar ist und so kalibriert ist, dass eine wirksame Dosis der Zusammensetzung zur Verwendung nach einem der Ansprüche 1 bis 11 oder der Formulierung zur Verwendung nach Anspruch 12 bei Aktivierung durch einen Anwender abgegeben wird.

## Revendications

1. Composition pour une utilisation dans le traitement ou la prévention d'une maladie ou d'une affection choisie dans le groupe constitué par la syncope, la réaction vasovagale chez les donneurs de sang, l'hypotension induite par la dialyse et l'hypotension orthostatique symptomatique, par une administration sublinguale, la composition comprenant :
- une quantité efficace par dose de capsaïcine ; et
- une quantité efficace par dose de caféine ;
- dans laquelle les quantités efficaces combinées de la capsaïcine et de la caféine sont efficaces pour prévenir la syncope, la réaction vasovagale chez les donneurs de sang, l'hypotension induite par la dialyse ou l'hypotension orthostatique symptomatique lorsqu'elles sont administrées par voie sublinguale à un sujet en ayant besoin ;
dans laquelle la composition comprend en outre une quantité efficace par dose d'un α-agoniste, de préférence dans laquelle l'α-agoniste est choisi dans le groupe constitué par la phényléphrine et l'étiléfrine (éthylphényléphrine), et de préférence dans laquelle la quantité efficace par dose de l'α-agoniste est comprise entre 1 et 100 mg, plus préférablement entre 2 et 70 mg, encore plus préférablement entre 5 et 50 mg.

2. Composition pour une utilisation selon la revendication 1, dans laquelle la quantité efficace par dose de capsaïcine est comprise entre 0,1 et 10 mg, de préférence entre 0,3 et 5 mg, plus préférablement entre 0,5 et 1,5 mg.

3. Composition pour une utilisation selon l'une quelconque des revendications 1 et 2, dans laquelle la quantité efficace par dose de caféine est comprise entre 50 et 400 mg, de préférence entre 100 et 300 mg, plus préférablement entre 150 et 250 mg.

4. Composition pour une utilisation selon l'une quelconque des revendications 1 à 3, comprenant en outre un ou plusieurs supports pharmaceutiquement acceptables, où le support pharmaceutiquement acceptable est compatible avec une administration sublinguale.

5. Composition pour une utilisation selon la revendication 4, dans laquelle le support pharmaceutiquement acceptable est choisi dans le groupe constitué par un support aqueux, un agent gélifiant et des combinaisons de ceux-ci.

6. Composition pour une utilisation selon la revendication 5, dans laquelle le support aqueux est choisi dans le groupe constitué par l'eau, un alcool et des combinaisons de ceux-ci.

7. Composition pour une utilisation selon la revendication 5, dans laquelle l'agent gélifiant est choisi dans le groupe constitué par la polyvinylpyrrolidone, le polyéthylèneglycol et des combinaisons de ceux-ci.

8. Composition pour une utilisation selon la revendication 7, dans laquelle le polyéthylèneglycol est choisi dans le groupe constitué par le polyéthylèneglycol 3350, le polyéthylèneglycol 400 et des combinaisons de ceux-ci.

9. Composition pour une utilisation selon l'une quelconque des revendications 1 à 8, dans laquelle la composition est une solution aqueuse.

10. Composition pour une utilisation selon l'une quelconque des revendications 1 à 9, dans laquelle la composition est un gel aqueux.

11. Composition pour une utilisation selon l'une quelconque des revendications 1 à 10, dans laquelle la composition comprend en outre un agent aromatisant et/ou un agent stabilisant, et/ou un agent colorant.

12. Formulation sublinguale d'administration de médicament pour une utilisation dans le traitement ou la prévention d'une maladie ou d'une affection choisie dans le groupe constitué par la syncope, la réaction vasovagale chez les donneurs de sang, l'hypotension induite par la dialyse et l'hypotension orthostatique symptomatique comprenant :
- la composition pour une utilisation selon l'une quelconque des revendications précédentes ;
- un ou plusieurs supports pharmaceutiquement acceptables compatibles avec une administration sublinguale.

13. Dispositif d'administration de médicament comprenant un réservoir contenant une ou plusieurs doses efficaces de la composition pour une utilisation selon l'une quelconque des revendications 1 à 11 ou de la formulation pour une utilisation selon la revendication 12.

14. Dispositif d'administration de médicament selon la revendication 13, dans lequel le dispositif peut être activé par un utilisateur et calibré pour administrer une dose efficace de la composition pour une utilisation selon l'une quelconque des revendications 1 à 11 ou de la formulation pour une utilisation selon la revendication 12 lors de l'activation par un utilisateur.
